# EUROPEAN PATENT SPECIFICATION

(11) **EP 3 126 813 B1**
(45) Date of publication and mention of the grant of the patent: **20.01.2021**
(21) Application number: 15712964.4
(22) Date of filing: 31.03.2015
(51) Int. Cl.: G01N 1/10, A61B 10/00, A61B 5/00, A61M 31/00, B01L 3/00, E21B 49/08, G01T 7/02, A61B 5/06, A61J 1/00, G01N 33/18, E21B 41/00, G01N 1/16, G01V 1/02

(54) **SYSTEM AND METHOD FOR ACQUIRING AT LEAST ONE SAMPLE FROM A FLUID**
SYSTEM UND VERFAHREN ZUR ERLANGUNG ZUMINDEST EINER PROBE EINER FLÜSSIGKEIT
SYSTÈME ET PROCÉDÉ POUR L'ACQUISITION D'AU MOINS UN ÉCHANTILLON D'UNE FLUIDE

(30) Priority: 03.04.2014 US 201414244283; 03.04.2014 US 201414244292
(43) Date of publication of application: 08.02.2017
(60) Divisional application: 16203352.6
(73) Proprietor: Fluidion, 75001 Paris (FR)
(72) Inventor: ANGELESCU, Dan, F-94170 Le Perreux sur Marne (FR); HAUSOT, Andreas, 75001 Paris (FR)
(74) Representative: Loyer & Abello
(86) International application number: PCT/EP2015/057126
(87) International publication number: WO 2015/150429

(56) References cited:
- WO-A1-2013/043737
- WO-A1-2014/004573
- GB-A- 2 252 296
- US-A- 3 729 855
- US-A- 5 058 674
- US-A1- 2003 092 393
- US-A1- 2007 193 377
- US-A1- 2011 314 936
- US-A1- 2013 269 423
- US-B1- 6 457 539

## Description

### Cross-Reference to Related Applications

### Technical Field

The present invention generally relates to an electrically passive device capable of communicating its position by acoustic emission at specific time intervals, and/or of retrieving and/or sensing/and/or determining characteristics of fluid samples at, for example, specific time intervals, and/or of time-release of particles, chemical products, or pharmaceutical products. More particular embodiments of the present invention relate, for example, to an electrically passive vessel for acquiring samples or releasing various particle/products in a subsurface formation (such as a geological or marine formation) or a living body, with the optional capability of providing measurements on the sample, and/or communicating its position via acoustic emissions.

### Background Art

Obtaining and analyzing samples of fluid from subsurface reservoir formations is often conducted during oil and gas exploration. Such operations are hindered by the harsh subterranean environment specific to oilfields, including high temperature and pressure (HPHT), corrosive fluids, and severely constrained geometry. The difficulty in acquiring and performing measurements on fluidic samples in such an environment are further complicated by use of electronic sensors that typically require power, monitoring and/or telemetry.

Several oil-field related operations, such as fracturing a geological formation, would greatly benefit from the capability of producing a map of the subterranean fracture geometry, and of the fracture evolution in time. Such capability does not currently exist. A similar need exists for a technology which can be used in monitoring and performing fracture analysis of subterranean carbon dioxide sequestration reservoirs.

Measurements of fluid properties and composition from an oil well are difficult to perform in the oilfield environment. The capability to inject very small sensing devices far into a geological formation by use of a Proppant or similar means of sensor transport, and to be able to determine their position and the precise moment when they perform a measurement or acquire a sample would greatly benefit the industry.

Measurements need to be performed in other types of situations, where the deployment of active sensing systems with on-board electronics and data transmission capabilities may either be impossible due to environmental issues (for example temperatures and pressures that are too high) or may prove to be too expensive to justify economically. Typical examples involve measurements within aquifers, potable water wells, or in a submarine environment. Such an environment may be a lake, or a sea or ocean. Still further environments include where or when there is a lack of power, such as in remote areas of the world.

The capability to perform viscosity measurements on fluid samples is extremely important in a variety of industries: chemical engineering, food industry, oilfield, to name a few. Usually this is accomplished using large laboratory instruments such as capillary viscometers and rheometers, or by using portable lighter weight instruments. In most cases, these instruments are operated using electrical power, and require sample manipulations that are difficult to automate. In some environments such instruments may be impractical due to their size (such as in difficult to reach areas, or within downhole oilfield tools), may be dangerous due to their electrical operation (inside explosive environments such as refinery facilities and tanks, near oil and gas wells), may be incompatible with the shocks or vibrations (within an oil well), or may be simply difficult to adapt. In this case new types of viscosity measurements need to be devised that avoid such inconveniences.

Samples often need to be acquired in explosive atmospheres (ATEX environments) such as inside refinery tanks (to determine the fluid quality and stratification), within refineries or gas plants or other facilities dealing with explosive environments. In such situations the sampling equipment should not pose a risk of generating an explosion, as would be the case if an electrical spark were created. All-mechanical systems that are ATEX-certified are currently used in the industry to perform this type of sampling.

Furthermore, samples may need to be acquired from fluids that are at high pressure, such as in a chemical factory, a refinery, inside an oil well, or at high depth within the ocean. When pressure is lowered, such samples may change (or degrade) by undergoing a thermodynamic phase transition leading to phase separation (i.e. gas may separate from oil, or asphaltenes may precipitate from heavy oil), in a possibly irreversible manner. Sample containers in this case may need to be filled slowly, at controlled inflow into the sample vial or chamber, in order to preserve the thermodynamic equilibrium and prevent, for example, a pressure shock that may lead to phase separation or other irreversible changes in the sample constituency. Such samples may also need to be maintained at high-pressure conditions subsequent to sample acquisition (for example during sample tool retrieval, or during transportation to a remote laboratory), to prevent sample degradation and maintain the sample characteristics unchanged.

Additionally, samples are often required in environments that pose objective risks and dangers, or that are physically remote, or where frequent sampling using manual devices may prove impractical: sites of nuclear disasters, military battlegrounds, biohazard or chemical hazard areas, terrorist attack sites, remote natural resources such as rivers and lakes, coastal waters and other offshore locations, deep water and sub-sea environments. In such cases, robotic equipment may need to be deployed, and the capability to take and analyze such samples may provide important information that cannot be acquired using the on-board inline sensors present on such equipment. Such equipment may include autonomous or remotely-operated vehicles or robots; remotely-operated underwater vehicles; autonomous underwater vehicles such as buoyancy-driven gliders and wave gliders; airborne or ground drones; other type of robotic equipment.

Samples often need to be acquired and analyzed to detect trace levels of certain contaminants that may be too dilute to enable direct detection. Sample pre-concentration may be required in such cases, by methods such as filtration using mechanical filters, polymeric filters, fiber glass filters, affinity columns, solid phase extraction columns, gas chromatography pre-concentration tubes and columns, or other types of material showing particular affinity for the contaminant, and possibly included in porous or packed form. Particular care needs to be taken when acquiring such samples to prevent scavenging of the sample by, or its adsorption to, the materials of the sampling vial or chamber, or of the transport tubes. The filter material may need to be backflushed, often with a different solution, to remove filter cake buildup and to generate a pre-concentrated sample that may need to be stored into a separate vial or container for transport, storage, or for further manipulation and/or analysis. Alternatively, the filter itself may be retrieved and analyzed, after applying an optional extraction protocol, using laboratory equipment such as gas chromatography, high precision liquid chromatography (HPLC), mass spectroscopy, gamma ray spectroscopy, or other analytical chemistry, biochemical, biological, or nuclear equipment, and possibly after solvent or thermal desorption.

Often there is a need to perform a chemical or biochemical reaction on the acquired sample, by bringing it in contact with a known quantity of chemical or biochemical reagent that will lead to a property change as a consequence of the reaction. The chemical or biochemical reagent may be present as a liquid, solid, powder, gel, gas, emulsion or foam, and it may be attached to, or immobilized on, a solid substrate such as the walls of a vial, a strip of metal, tissue, plastic, paper (as is the case of colorimetric test strips). The reagent may be lyophilized. Often, the (bio)chemical reaction results in color change, which can be detected optically by spectrophotometric absorbance or by colorimetry. Other times direct fluorescence of the sample, or the presence of a fluorescent byproduct, can be detected by fluorescence measurements. Many other properties of the sample may be measured such as (without limitation) optical absorbance, chemiluminescence, color, turbidity, fluorescence intensity, index of refraction, conductivity, density, viscosity. In some cases the usage of a microplate with multiple wells may be necessary to prepare a sample and perform measurements on it.

Certain chemical or biochemical sample preparation protocols may require more complex sample manipulations, such as reaction with a first reagent, waiting for an incubation or reaction time, and then bringing the sample in contact with a second reagent. This sequence may need to be repeated several times.

For pollution monitoring, often samples are not acquired instantaneously but rather over longer periods of time. The rate of sample intake may be proportional to flow velocity in the body of water being sampled. This technique provides "integrated" water samples, which allow in some circumstances to determine the average pollution of the body of water over a period of time, rather than instantaneous pollution.

In certain applications, the moment when a sample needs to be acquired may not be known in advance, so that a pre-programmed sampling sequence may not be appropriate. In this case, individual control of the moment when each sample is performed may be required. For example, samples may need to be acquired in the aftermath of an unforeseen accident, or as directed by an external signal.

In some cases, there may be a need to acquire a larger number of samples than the capabilities of a single sampling device. In this case, multiple sampling devices may need to be used in a "daisy chain configuration", in such a way that once a device acquires its maximum number of samples, it automatically triggers the next device that will take over the sampling tasks. Using this approach, the total number of samples that can be acquired is not limited by the capabilities of an individual device.

Often there is a need for injecting, or liberating, small particles or small amounts of chemicals at predefined times into a remote environment, or into an environment which is difficult to access. Such small particles or chemicals may be used as tracers, may participate in chemical reactions, or may be used as pharmaceuticals. Exemplary environments where such particles, chemicals, or pharmaceuticals may be injected include without limitation oil and water reservoirs, pre-existing or induced fractures within such reservoirs or within other geological formations, oil, water and/or gas wells, water bodies such as lakes, rivers and oceans, or a human body. It may be desired that such particles or chemicals react in an aggressive way with the fluid present in the remote environment, for example by producing an explosion or producing a rapid release of energy

Monitoring of hazardous waste disposal reservoirs and of adjacent aquifers for contamination mapping and leaching is also a very important domain, where the need for miniaturized and economical sensing solutions is prominent.

Unintended leaks, spills or discharges occur frequently around industrial sites, and may affect environments such as the deep ocean (off-shore environment), a river, a lake, an estuary, the coastal water, or the atmosphere. In the case of an event concerning an unintended discharge, leak, nuclear or chemical spill from an industrial site or other facility, there is an inherent desire within the industry to understand the impacts of that event on the environment. In addition, it would be desirable to be able to gather accurate data concerning the distribution and movement of the pollution over time as it passes through the environment.

There are many factors that may affect the complex interaction of a leak into a marine environment, including ocean currents, wind, waves, thermoclines, buoyancy, pressure, formation of gas hydrates and phase transitions just to name a few. In order to make the best possible predictions using 3 dimensional models (in space) and 4 dimensional models (over time) as an off-shore leak progresses, real data points are required to be entered into the models. Samples taken at different points and different times around the spill area are a valuable method for determining the progression of a spill through the environment. However, technology today is very limited in terms of deployment and ability to acquire a significant number of samples at multiple depth points, at multiple radial points from the source and at multiple points in time. Sample acquisition systems may need to be deployed in a way that is non-intrusive to the normal operations in and around the industrial facility (which may be a drilling rig, an off-shore platform, a ship, a tanker, a pipe etc.), both on the ocean surface and in the subsea. In the early hours and days after an event occurs, resources are normally dedicated to critical tasks other than environmental monitoring or sampling. Additionally, in most cases, the sampling capability is also not located at the industrial site, but more likely on-shore and may take days for the equipment to reach the offshore facility. There is a need for a system that is put in place around an industrial facility as a precautionary measure at an early stage in the project. Furthermore, the position of the system should be accurately determined at the time of installation. Acquired samples and subsea systems also need to be easily retrievable by an ocean vessel at the ocean surface.

In the case that a sampling system is placed in the marine environment for an extended period of time, biofouling and biogrowth tends to develop on the system, including at the sampling intake ports. Similarly, in a deep-water environment near hydrocarbon production facilities, there is a risk of methane hydrate formation. This provides a serious risk of blockage, contamination and/or unrepresentative sampling. Thus the need for a system that is able to not only remain in standby mode ready for deployment, but also be in a protected environment that rejects any biogrowth or methane hydrate from forming during the standby period.

Often there are cases where a sampling unit or an array of sampling units needs to be deployed in a non-static environment, such as when there is a natural ocean current or wave motion due to winds, or other movement within the water column. This movement may, or may not, be in the same direction at all times, may not be predictable, and may cause the sampling unit or string of units to be in a position that is not vertically above the original anchoring point, or vertically below a buoy or similar water-surface mooring point. Consequently, this may cause error in the assumed position data associated with the sampling unit or array.

Currently, sample loading and measurement protocols for microbiological measurements often need to be performed manually, requiring specialized labor and equipment and incurring significant costs. In addition, such measurements require the collection of a representative sample from the environment being monitored (that may be a lake, a river, a pool, a reservoir, a groundwater well, an estuary, coastal water, drinking water, wastewater on any other water source), and transportation to a specialized laboratory where the measurements are performed. This protocol provides a risk of human error in the sampling and/or transportation procedure, leading to an unrepresentative sample being collected, or to sample contamination and/or degradation during transport. There is a need therefore for a device that is capable to perform the sampling and analysis operation in-situ, in a repeatable and reproducible manner, thus bypassing certain human intervention steps that are prone to creating errors.

In certain applications, there is a need for very precisely controlling the moment when each sample is being acquired in a series of sampling operations. These moments may depend of certain external events that cannot be predicted or anticipated at sampler installation, and therefore the sample acquisition moments cannot be pre-programmed in the sampler design.

A sampler may also require a means to actuate the sampling mechanism in a rapid manner upon the receipt of the signal to sample following external trigger activation, such as in the seconds or milliseconds following the receipt of the signal. Furthermore, a sampling device may require means for confirming that a sample was actually acquired, for recording the exact time when each sample was acquired (also known as a timestamp), as well as a certain amount of information corresponding to the sampling process, such as the total duration over which a sample container was filled, the total amount that was sampled etc.

It is important that any timing mechanism used to trigger the acquisition of a fluid sample be accurate, however in certain applications such timing may not prove accurate enough. For example, timing errors in fluidic clocks may occur due to small manufacturing imperfections that may change the overall timing of the sample acquisition. For example, a ten percent relative error in the volume of a timing cavity will result in a ten percent relative error in the timing of that particular sample acquisition. If the overall timing was designed to be ten hours, a ten percent error will introduce a one-hour absolute error in the timing, so the sample may be acquired an hour prior to, or an hour later than the scheduled time.

Such timing errors provided by fluidic clocks may become particularly problematic when a series of samples needs to be acquired in sequence. For example, assume one sample needs to be acquired every hour for a period of twenty four hours. Twenty four sampling devices are deployed at t=0, device numbered n (1<n<24) having a time constant of n hours prior to triggering the acquisition of its corresponding sample. If there is a ten percent error in the fluidic clock of each sampling device, that means that it is likely that the order of the sampling events will be disturbed. For example, the 10th device may acquire its sample at t=11h, and the 11th device at t=10h, thus they will be out of order. US2013/269423 discloses a sampling device comprising an isolated cavity, an electrically passive timing mechanism, a mechanical structure separating the isolated cavity from the exterior environment, such that at the end of a timing interval the timing mechanism acts on the mechanical structure to rupture and/ or collapse the mechanical structure, thus bringing the isolated cavity in contact with the external fluid. The electrically passive timing mechanism includes: a timing diaphragm; a timing cavity; and a conduit in fluidic communication with the timing cavity, such that upon applying pressure to a timing fluid within the conduit, said timing fluid advances within the conduit and upon reaching the timing cavity and filling it after the timing interval, the timing fluid applies pressure to the timing diaphragm which ruptures and/or collapses the mechanical structure thus allowing external fluid to enter the isolated cavity.

US 2011/314936 A1 and GB 2 252 296 A disclose further sampling devices.

### Summary of the Invention

The invention refers to a system for acquiring at least one sample from a fluid as disclosed in claim 1, and a corresponding method as disclosed in claim 13. Advantageous options are disclosed in the dependent claims.

### Brief Description of the Drawings

The foregoing features of the invention will be more readily understood by reference to the following detailed description, taken with reference to the accompanying drawings.
Fig. 1 shows deployment of a device for use in sampling hydrocarbons during fracturing or fluid injection operations, in accordance with an embodiment of the invention.
Figs. 2(a-d) show the device of Fig. 1 in more detail, in accordance with an embodiment of the invention. Fig. 2(a) shows the device prior to activation. Fig. 2(b) shows the device with the isolation membrane collapsed. Fig. 2(c) shows the device with the sample chamber filled with sample fluid. Fig. 2(d) shows the device ready to be interrogated after surface retrieval.
Fig. 3(a) shows a burst of acoustic energy resulting from the rupturing of an isolation membrane, in accordance with an embodiment of the invention. Fig. 3(b) shows multiple microphones placed at different positions in the formation, for recording the arrival time of the wavefronts caused by ruptured isolation membranes, in accordance with an embodiment of the invention.
Fig. 4 shows a passive timing device that includes a pharmaceutical product for release within a human body, in accordance with an embodiment of the invention.
Fig. 5 shows a passive timing device that includes a filter for containing the broken diaphragm particles, in accordance with an embodiment of the invention.
Fig. 6 shows integration of a plurality of sampling devices and/or mechanisms within an oilfield-sampling tool, in accordance with an embodiment of the invention.
Fig. 7 shows an array of smart sampling devices embedded within an underwater measurement system which may be attached with a cable to either a buoy, a rig, a vessel or a ship, in accordance with an embodiment of the invention.
Fig. 8A shows a viscosity measurement system that may be fully passive, in accordance with an embodiment of the invention. Fig. 8B shows another viscosity-measurement system, in accordance with an embodiment of the invention.
Fig. 9A shows a sampling and measurement system that includes a manifold, in accordance with an embodiment of the invention. Fig. 9B shows an external sampling conduit connected via a T-junction to a reagent reservoir, in accordance with an embodiment of the invention.
Fig. 10A shows a device that may be used to acquire a sample from a contaminated external fluid, in accordance with an embodiment of the invention. Fig. 10B shows the device of Fig. 10A after sampling, in accordance with an embodiment of the invention.
Figs. 11A-C shows a system capable of acquiring a sample from an external fluid, and maintaining it at a desired pressure for extended periods of time, in accordance with an embodiment of the invention. Fig. 11A shows the system prior to acquiring a sample. Fig. 11B shows the system after acquiring the sample. Fig. 11C shows the system after sample pressurization.
Fig. 12A shows a device for performing a sampling operation from a high-pressure external fluid without shocking the fluid, in accordance with an embodiment of the invention. Fig. 12B shows the device of Fig. 12A that further allows the sample to be maintained at a high pressure after sampling, in accordance with an embodiment of the invention.

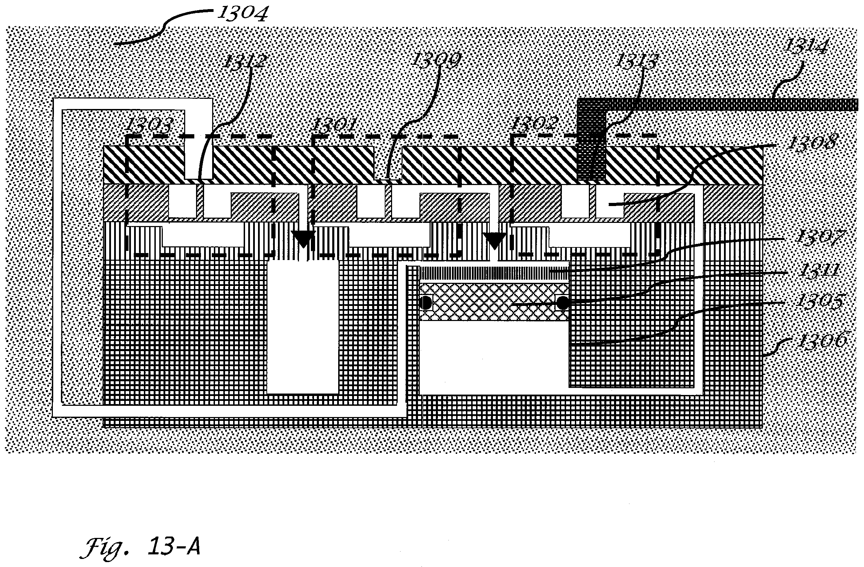

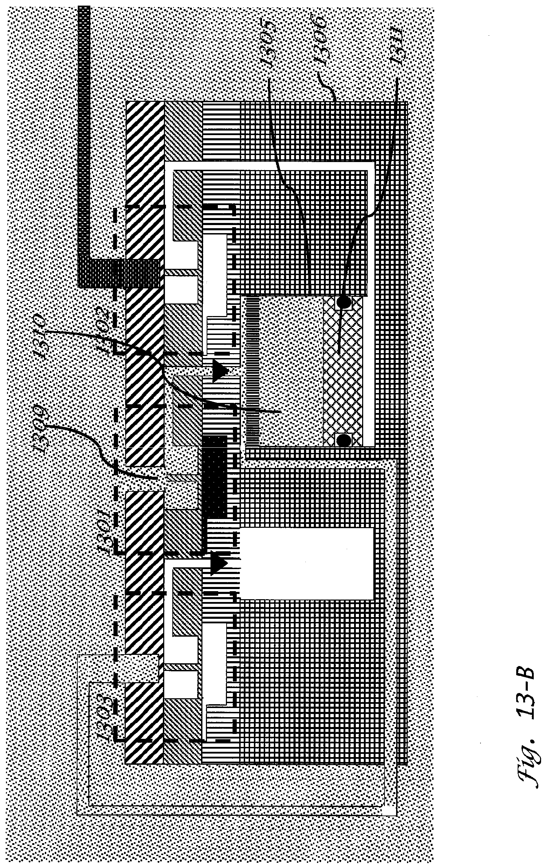

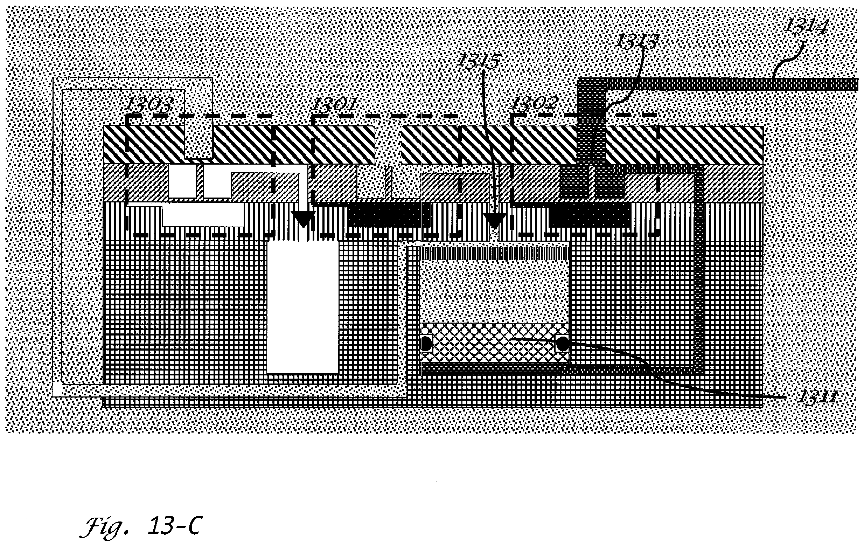

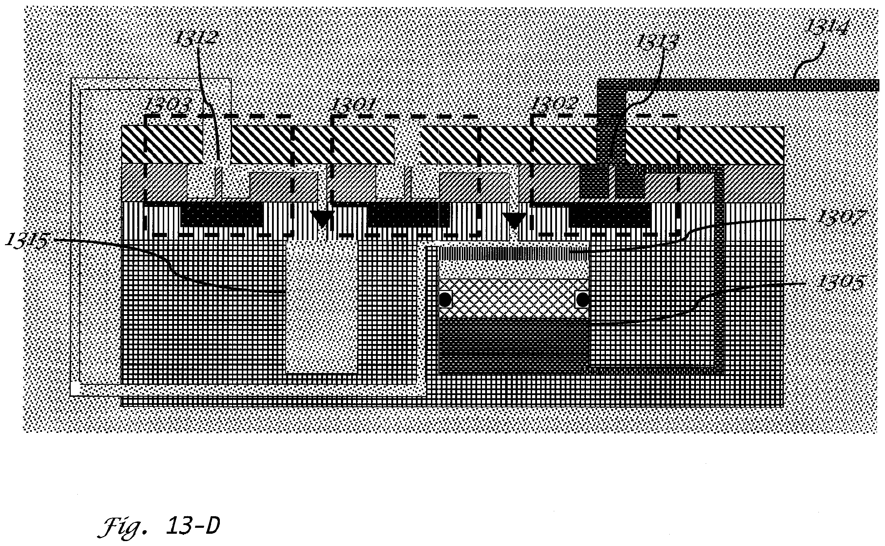

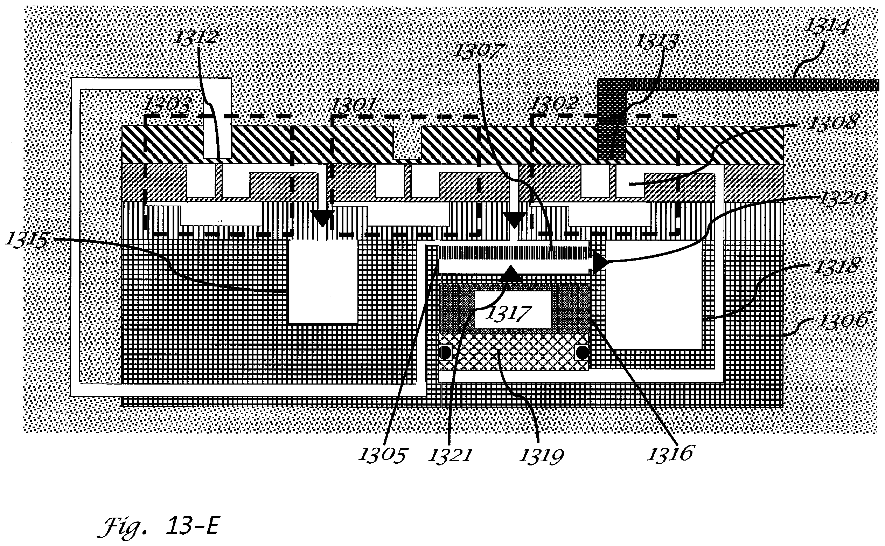

Figs. 13A-D show, in chronological order, operations performed by a system that, in addition to sampling at a time controlled by a passive timing mechanism, integrates a mechanism allowing the subsequent transfer of the sample from an initial sampling chamber to another sampling chamber after a given amount of time, in accordance with an embodiment of the invention. Fig. 13A shows the system prior to acquiring a sample. Fig. 13B shows the system after collapse of a first device's mechanical structure. Fig. 13C shows the system after the later collapse of a second device's mechanical structure. Fig. 13D shows the system after the later collapse of a third device's mechanical structure. Fig. 13E shows the system of Figs. 13A-D modified such that the sampling chamber 1305 including a first reservoir and a second reservoir.
Fig. 14 shows multiple sampling systems connected in a "Daisy-chain" configuration, in accordance with an embodiment of the invention.
Fig. 15 shows a system capable of controlling the sampling times of a plurality of sampling devices using a trigger device, in accordance with an embodiment of the invention.
Fig. 16(a) shows a sampling device that uses a piston in place of a timing diaphragm, and includes an optional triggering system, in accordance with an embodiment of the invention. Fig. 16(b) shows the sampling device from Fig. 16A after the piston has pierced the mechanical structure, thus enabling the acquisition of a sample, in accordance with an embodiment of the invention.
Fig. 17 shows a sampling device that includes an optional triggering system, which performs a sample acquisition within a given time interval, in accordance with an embodiment of the invention.
Fig. 18 shows a sampling device similar to that embodiment shown in in Fig. 17, but with the timing mechanism modified in such a way that the sample acquisition is performed in a much shorter time interval, in accordance with an embodiment of the invention.
Fig. 19 shows a sampling device consisting of two sampling mechanisms whereas the timing of the two sampling mechanisms is different due to their different timing cavity volume, in accordance with an embodiment of the invention.
Fig. 20 shows the installation of a sampling system by a deployment vessel, in accordance with an embodiment of the invention. The installation requires accurate positioning of the system on the sea floor and may optionally be aided by an ROV for disconnection from the surface tether and/or accurate placement relative to the wellhead.
Fig. 21 shows an array of sampling systems that are installed in standby mode around an oil rig or offshore platform, in accordance with an embodiment of the invention. At the surface facility, an acoustic transmitter/receiver continuously or at pre-defined time intervals transmits an acoustic signal. This signal can be detected by the sampling systems, which indicates normal operation on surface, thus the systems will not deploy and will remain in standby mode.
Fig. 22 shows the embodiment of Fig. 21 where there is a loss of transmitted signal from the surface facility, in accordance with an embodiment of the invention. This may be indicative of an abnormal situation at surface. The systems may continue to attempt detection of the acoustic source from surface for a period of time. If there is a continued period of loss of transmitted signal, the sampling systems will be triggered to deploy and commence monitoring.
Fig. 23 shows sampling arrays after deployment due to loss of transmitted signal from the surface facility, in accordance with an embodiment of the invention. Upon deployment of the sampling arrays, each sample device may automatically start sample acquisition at predefined timing intervals.
Fig. 24 shows how a specific configuration of the sampling arrays may be varied according to the location relative to the wellhead, in accordance with an embodiment of the invention. This may serve to effectively increase sampling resolution.
Fig. 25 shows the effect that ocean currents or other movement in the water column may have on the positioning of the sampling arrays, and how compensation using accelerometers, relative bearing meters or similar devices may be used to accurately determine the position of each point in the sampling array over time, in accordance with an embodiment of the invention.
Fig. 26 shows the sampling systems after sampling has been completed and several options for retrieval of the sampling string and/or sampling system on the surface, in accordance with an embodiment of the invention.
Fig. 27(a) shows an override signal being emitted by a transmitter at the surface and detected by the sampling systems, in accordance with an embodiment of the invention. Fig. 27(b) shows the override signal activating a buoyancy device to carry a retrieval tether to the surface for subsequent retrieval of the unactivated sampling systems from a body of water, in accordance with an embodiment of the invention.
Fig. 28 shows a sampling system in standby mode having an additional buoyancy device which remains at ocean surface level, and includes a data transmitter/receiver device, so that when a trigger signal is received, the device is able to send a deployment command via a cable to trigger the sampling system and/or release the sampling deployment buoyancy device, in accordance with an embodiment of the invention.
Fig. 29 shows additional sampling systems (or rapid deployment kits) which may be deployed at a greater perimeter from the wellhead after a pollution event has occurred for additional leak monitoring, in accordance with an embodiment of the invention.
Fig. 30 shows details of a sampling system, in accordance with an embodiment of the invention.
Fig. 31 shows a timestamping and sample acquisition monitoring system that allows the determination of the timestamp of each sample acquisition and of different other parameters related to the sample acquisition process based on measurements of pressure levels within the sample chamber and, optionally, in the external fluid, in accordance with an embodiment of the invention.
Fig. 32 shows a sampling device implementing a passive timing mechanism allowing the timing of a sample acquisition to be triggered by the acquisition of the previous sample, in accordance with an embodiment of the invention.
Figure 33 shows sampling devices described in the embodiment of Fig. 32 connected together so as to trigger each other, in accordance with an embodiment of the invention.
Figure 34 shows sampling devices described in the embodiment of Fig. 32 connected together so as to trigger each other, further shows a manifold, in accordance with an embodiment of the invention.

### Detailed Description of Specific Embodiments

In illustrative embodiments, an electrically passive device and method for in-situ acoustic emission, and/or releasing, sampling and/or measuring of a fluid or various material(s) is provided. The device may provide a robust timing mechanism to release, sample and/or perform measurements on a predefined schedule, and, in various embodiments, emits an acoustic signal sequence(s) that may be used for triangulation of the device position within, for example, a hydrocarbon reservoir or a living body. Details are discussed below.

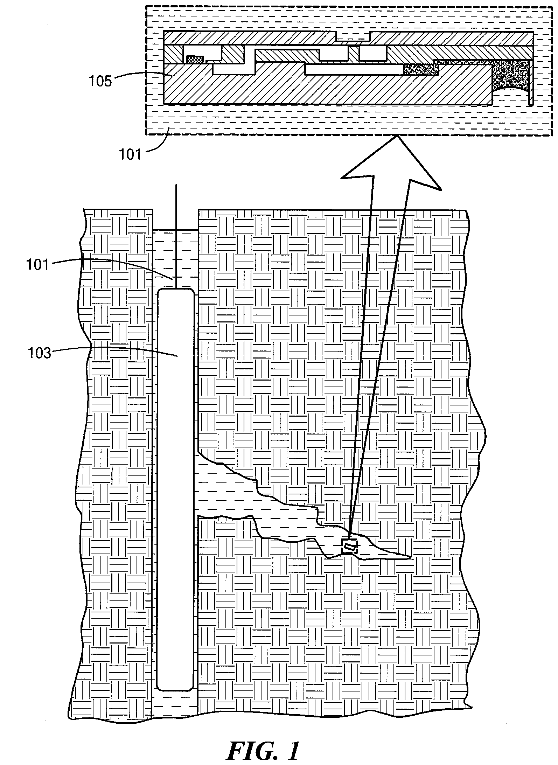

Fig. 1 shows deployment of a device **105** for use in sampling hydrocarbons during fracturing or fluid injection operations, in accordance with an embodiment of the invention. It should be noted that discussion of the specific device **105** shown, for use in sampling hydrocarbons, is for illustrative purposes only. Other device configurations and applications are within the scope of the present invention. For example, the device **105** may be deployed in a wide range of environments including, without limitation, within a pipe, a well, an engine, a hydrocarbon reservoir, an aquifer, a body of water, an oil field tool, a waste disposal reservoir, a proppant formulation and a living body to release, sample or measure various fluids (including a gas) or other material(s).

The device **105** may be deployed, without limitation, in downhole fluid 101 within a fracture in an underground formation. The device may be, for example, pumped or otherwise injected, into the rock matrix. The device **105** may work in combination with conventional oilfield measurement tools **103** or autonomous battery-operated sensors, that may be placed in the well in hydraulic communication with the fracture where the device **105** is injected. The device **105** may be used at very high pressures or temperatures, thus providing a pathway to performing measurements within wells which are currently inaccessible to existing sensor technology due to, without limitation, severely constrained geometry, corrosive fluids, elevated pressure and/or temperature. Examples of adverse well environments include recently developed deep-sea well reservoirs in the Gulf of Mexico. The device **105** may be used in areas with no available power. The device **105** may be used in explosive environments or atmospheres, where electric equipment poses a risk of explosion. The device **105** may be used for water and/or air quality monitoring in and around cities, chemical plants, nuclear sites, offshore platforms and other oilfield structures, military missions and battlegrounds. The device **105** may be used in robots such as marine remotely-operated underwater vehicles, autonomous underwater vehicles, airborne or ground drones and vehicles, and other types of robotic equipment. The device **105** may be used where and/or when there is no power available, such as in certain remote area.

The device may be of any size, dependent for example, on the application. In various embodiments, the device may be fabricated, at least in part, using micromachining or micro system technology, using, for example, silicon, glass and/or ceramics. In various embodiments, certain portions of the device may not be included in the micromachined process, such as the sampling chamber (described in more detail below), which may be, for example, a separate vial or other container.

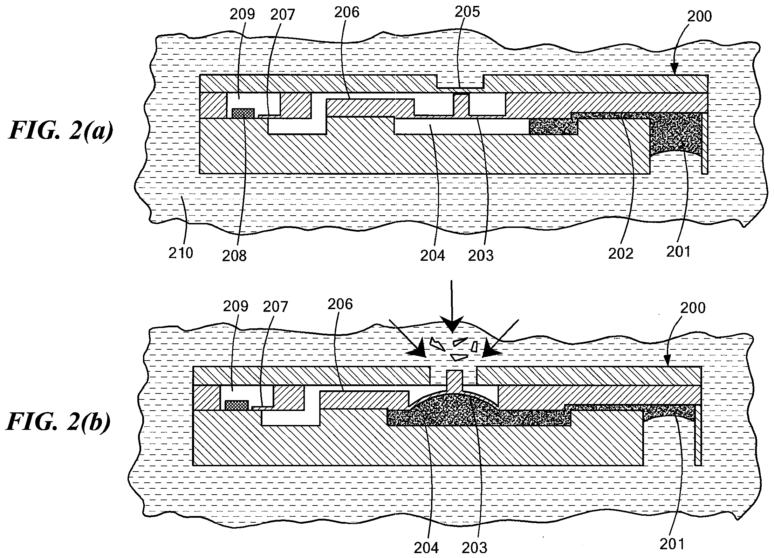

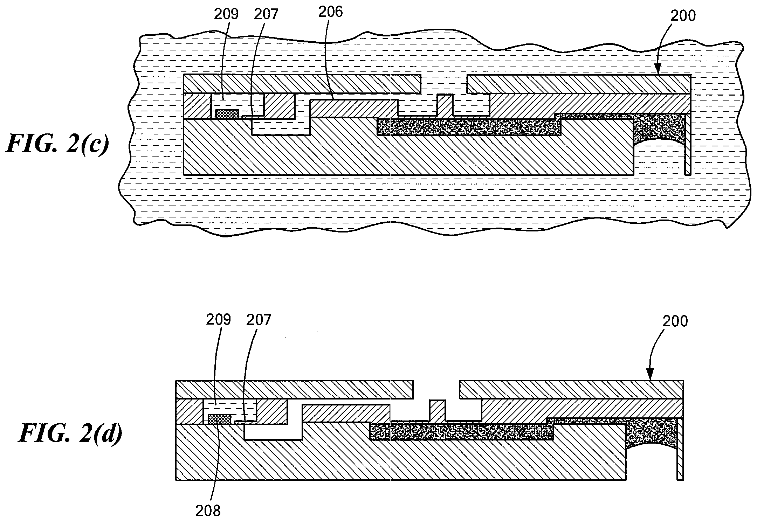

Figs. 2(a-d) show the device in more detail, in accordance with various embodiments of the invention. Fig. 2(a) shows the device **200** prior to activation. The device **200** includes at least one sampling mechanism for obtaining a sample of the external oil-well fluid **210.**

In illustrative embodiments, the sampling mechanism includes a microfluidic timing mechanism for obtaining the fluidic sample. More particularly, the microfluidic timing mechanism may include a conduit that may be a microfluidic channel **202** or a capillary tube. The conduit may be partially filled with a timing fluid **201** (in other embodiments, the timing fluid may be without limitation, the external fluid that enters from the isolated cavity 206, described in more detail below). Capillary trapped timing fluid **201** may initially be held in place within the microfluidic channel by, without limitation, surface tension. The microfluidic channel **202** leads to a timing cavity **204** that may be of known volume. The timing cavity **204** may initially be, without limitation, empty.

Upon applying pressure to the timing fluid **201**, the timing fluid **201** advances within the microfluidic channel **202** into the timing cavity **204** such that it causes a mechanical structure **205** to rupture (and/or collapse) after a time delay. The mechanical structure 205 may be insoluble in the environment or fluid in contact with the device. The mechanical structure **205** may be insoluble in water, bodily fluids, oil, oil field fluid, crude oil, salt water, or sea water, or combinations thereof. The mechanical structure **205** may be made of an inorganic material, a non-polymeric material, silicon, glass, or a ceramic, or combinations thereof. As used in this description and the accompanying claims, the term "inorganic" shall have the meaning indicated, unless the context otherwise requires: a material composed of atoms or molecules not containing carbon with, the exception of certain forms of carbon such as graphene, diamond, nanotubes and bucky-balls, which shall be considered inorganic. Examples of inorganic materials include, without limitation, all metals, all types of glasses, silicon compounds such as oxides and nitrides, ceramic materials, silicon (in all crystalline forms), quartz, diamond, sapphire, ruby, as well as all materials of geologic origin.

In accordance with various embodiments, the timing fluid may be routed, prior to entering the timing channel, through a trigger device that can enable or disable the passage of timing fluid as desired. The trigger device may be one of a check valve, an electrically-controlled solenoid valve, a fluidic switch, or any other type of active valve known in the art. Examples of different types of valves used in microfluidic devices are provided in "Components for integrated poly(dimethylsiloxane) micro fluidic systems" Electrophoresis 2002, 23, 3461-3473; "Micro Total Analysis Systems: Latest Achievements" Anal. Chem. 2008, 80, 4403-4419. The trigger device may also include a one-shot valve that initially blocks the passage of timing fluid and upon receipt of an external signal permanently opens the passage of timing fluid without requiring further power.

Prior to rupturing, the mechanical structure **205** isolates an isolated cavity **206**, which may include a sample chamber **209**, from the external environment, which may include an external fluid (which may be a gas). The mechanical structure **205** may be, without limitation, an isolation diaphragm or isolation membrane that provides a barrier from the external environment. An example of a delayed actuator with a visco-elastic timer is described in U.S. Patent 4,791,251 (Carter et al.).

Illustratively, the timing fluid **201** entering the timing cavity **204** may cause a timing diaphragm **203** to deflect. A piercing structure, such as a protrusion or other shaped structure on the timing diaphragm **203,** may then rupture or otherwise pierce the mechanical structure **205.** Various other membrane rupture mechanisms known in the art of micro fluidic systems, such as in systems used to provide drug encapsulation and delivery, may be utilized (see, for example, M.Staples et al.: Pharm.Res.,23,847 (2006); J.T. Santini et al.: Angew. Chem. Int. Ed. 39, 2396 (2000); J.H.Prescott et al.: Nat. Biotech. 24, 437 (2006), US Patent US7455667 (B2).

Fig. 2(b) shows the device **200** with the mechanical structure **205** collapsed after applying pressure to the timing fluid **201** (and after the time delay). The collapse of the mechanical structure **205** allows external downhole fluid to enter a sample chamber **209** via an isolated cavity/communication channel **206**. The sample chamber **209** may be prevacuumed or hold a gas prior to deployment of the device **200.** A particle filter may be placed within the isolated cavity/communication channel **206** to filter any contaminants. Note that prior to collapse of the mechanical structure **205**, the isolated cavity/communication channel **206** is typically inaccessible to the exterior environment. In other embodiments, the mechanical structure **205** may allow partial/filtered access to the isolated cavity/communication channel **206** prior to its collapse.

Fig. 2(c) shows the device **200** with the sample chamber **209** filled with sample fluid. An integrated one-way valve **207** (i.e., a check valve), may assure sample isolation from the external environment. An example of a micro-fabricated one-way valve is described in the following documents: S.Beeby, G.Ensel, M.Kraft: MEMS Mechanical Sensors, Artech House, Boston MA (2004); and K.W.Oh et al.: J. Micromech. Microeng.,16,R13-R39 (2006).

The timing mechanism, the sampling mechanism, and/or in various embodiments, the entire device, may be electrically passive such that it does not include any powered electronic components (e.g., an electronic power source, transmitter, amplifier etc...). In various embodiments, the timing mechanism, the sampling mechanism, and/or the entire device may be void of any active or passive electronic components.

The passive microfluidic timing mechanism may be based, at least in part, on the fact that the flow rate *f* of a Newtonian fluid through a capillary of roughly circular cross-section is proportional to the difference in pressure *ΔP* between the ends of the capillary multiplied by the fourth power of the hydraulic radius *R*, and is inversely proportional to the viscosity of the fluid *η* multiplied by the length of the capillary *l: f=π·ΔP·R⁴*/*(8·η·l).* In other embodiments, if the capillary is chosen to have a rectangular cross-section with width w and height *h<w,* the flowrate *f* can be calculated with the approximate formula: *f*=(*l-0.63h*/*w)·ΔP·w·h³*/*(12·η·l).* Such formulae may be found in the literature, for example in the following documents: Stone, H., Stroock, A., and Ajdari, A., "Engineering Flows in Small Devices," Annual Review of Fluid Mechanics, Vol. 36, 2004, p. 381 and D.E. Angelescu: "Highly Integrated Microfluidics Design", Artech House, Norwood MA USA (2011).

If an empty cavity of known volume (i.e., the timing cavity **204**) is separated from a high-pressure fluid by a capillary of appropriate geometry, the time required to fill the timing cavity **204** can be accurately determined from knowledge of device geometry, fluid viscosity and pressure differential. Assuming the timing fluid **201** has known characteristics, and that the pressure/temperature history is recorded, the filling time of the timing cavity **204** can be fully determined by geometrical device parameters such as timing cavity **204** volume, microfluidic channel **202** capillary diameter and length; the fourth power dependence on diameter allows control of the fill-up time over several decades, resulting in a very versatile timing mechanism. A fully characterized timing fluid **201** may be used that advantageously may be immiscible with both hydrocarbons and water. Examples of such timing fluids include, without limitation, various silicone oils and fluorinated solvents.

Alternatively, a non-Newtonian fluid with known rheological properties can be used as a timing fluid. In one embodiment, one may use a shear-thinning fluid as a timing fluid, which will result in a flowrate which is very low at low pressures, but increases significantly once the ambient pressure (and hence the shear stress in the microchannel) reaches a certain threshold value. In another embodiment, the timing fluid may be a visco-elastic fluid which behaves as an elastic body at low shear stresses, thus completely blocking flow at low pressures. As the pressure reaches a threshold value (corresponding to the yield stress of the timing fluid), the timing fluid will start flowing. This embodiment allows the passive timing devices described above to be inactive below a certain threshold pressure, thus allowing prolonged storage at a pressure situated below the threshold pressure.

Fig. 2(d) shows the device **200** ready to be interrogated after surface retrieval. The sample fluid stored in the sample chamber **209** remains isolated from the environment by the one-way valve **207,** so that various physical and chemical property measurements can be obtained. A sensor may be positioned within, or otherwise operationally coupled to, the sample chamber **209** and/or isolated cavity **206,** so as to provide various indications or measurements associated with the sample fluid. In various embodiments, a microelectromechanical sensor (MEMS) design may provide hermetic encapsulation of sensor components within, for example, the sample chamber **209**. The sensor may include a material that chemically reacts with the fluid, and/or an electrode allowing an electrochemical measurement to be performed on the fluid sample.

The above-described timing mechanism in conjunction with passive actuators may thus be used to deploy self-triggering sample acquisition devices/vessels. For deployment within a rock matrix, such devices may be density-matched to an injection fluid by incorporating vacuum cavities of appropriate dimensions, which will facilitate passive deployment by injection as well as device retrieval.

### Acoustic Emission and Triangulation

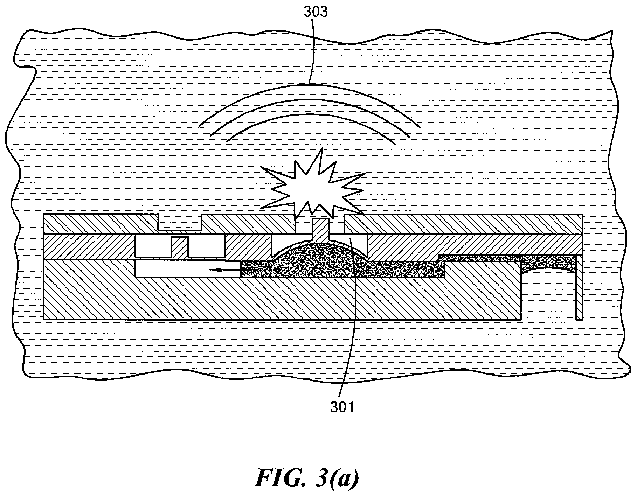

The above-described device for sample acquisition may be used to generate acoustic signals. For example, in various embodiments the timing mechanism may trigger the piercing of multiple mechanical structures/isolation diaphragms, possibly in sequence. For example, if the cavity behind each isolation diaphragm has volume V (initially under vacuum), upon piercing, these cavities will suddenly collapse and/or rupture, and fill with reservoir fluid at the ambient hydrostatic pressure. The filling of the empty cavity **301** may be very sudden, and will emit a very short burst of acoustic energy **303**, as shown in Fig. 3(a), in accordance with an embodiment of the invention. Laboratory studies of collapsing bubbles have been performed by others (for example, A. VOGEL, W. LAUTERBORN, R. TIMM: " Optical and acoustic investigations of the dynamics of laser-produced cavitation bubbles near a solid boundary", J. Fluid Mech., Vol. 206, pp. 299-338 (1989)), proving that the majority of the bubble energy is emitted into the acoustic transients. The total amount of energy that may be released by sudden filling of a cavity may be roughly estimated as E=pV, where p is the reservoir pressure. For an exemplary volume of 1 mm³ and an ambient pressure of 1000 Bar (app. 14500psi), this corresponds, without limitation, to an emission energy of 100 mJ in a time interval of approximately a fraction of a thousandth of a second to a few thousandths of a second. This corresponds to an acoustic power of over 10-1000W during each collapse event. Such acoustic emission can then be detected and recorded using remote microphones, hydrophones, geophones, accelerometers or other types of sensors or recorders.

The timing mechanism may trigger several acoustic events in sequence, with the time delay between consecutive collapses defined by the geometry of the associated micro fluidic channel and timing cavity. Each device and/or sampling mechanism may be built with a different timing sequence, or with different geometrical parameters, to provide a unique acoustic signature. Such devices may also be realized without a sampling cavity, with the sole purpose of emitting a sound at a time determined by the micro fluidic timing mechanism.

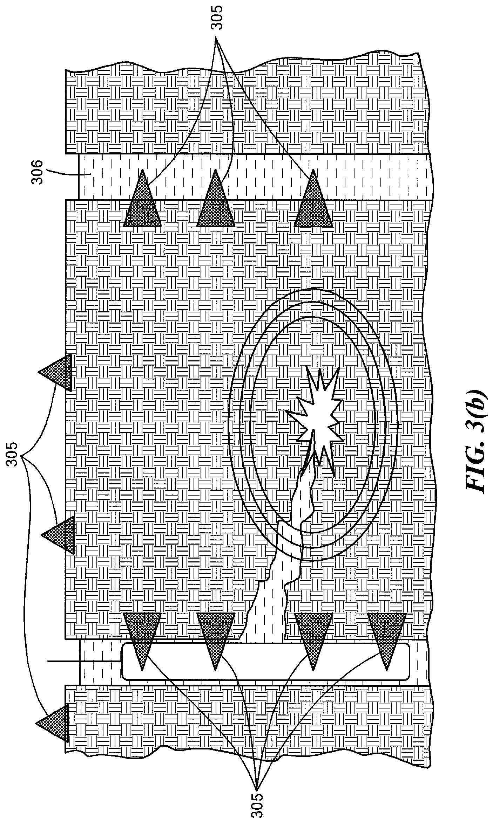

The acoustic emission for each collapse event will create an acoustic wavefront **303** which will propagate through the fluid and the surrounding rock matrix. The velocity of the wavefront will typically be equal to the sound velocity in the fluid, or in the rock matrix. By placing multiple microphones **305** at different positions in the formation, as shown, for example, in Fig. 3(b), the arrival time of the wavefronts at each microphone **305** may be determined. Based on the time delays between the arrival of the acoustic signal at the different microphones, combined with a knowledge or an educated estimation of the sound velocity in the medium, the position of the smart vessel can then be determined, using, without limitation, triangulation, similar to an underground GPS system, or using compressional / shear signal processing. The time of the sample acquisition may also be recorded. It is noted that Fig. 3(b) is by no way limited to the shown configuration of microphones or devices. In other embodiments of this invention, additional microphones may be located on the ground around the well, or at other subterranean locations, such as in a nearby well **306**, cavities, or holes.

### Usage as Vehicle for Time-release of Particles, Chemical products, or Pharmaceutical Products

The above-described devices may be used as vehicles for transport and time-release of, without limitation, micro- and nano-particles, chemical and/or pharmaceutical products, by including the products or particles within the isolated cavity and/or sampling chamber separated by the mechanical structure (e.g., isolation diaphragm). The timing mechanism may trigger the piercing of the isolation diaphragm after a time delay as described above, at which point the fluid surrounding the device penetrates within the cavity behind the isolation diaphragm and comes in contact with the particles, chemical and/or pharmaceutical products. The particles or products may then dissolve within, or mix with the fluid surrounding the device, thus releasing said particles or chemical or pharmaceutical products into the surrounding environment.

Said particles or chemical products or pharmaceutical products may include, without limitation, chemicals for sanitizing water or other fluids; fluorescent chemicals that may be used as flow tracers; various chemical reagents and chemical cleaning agents; pharmaceutical products such as medications or drugs; various types of nutrients; micro- or nano-particles to be used as flow tracers; materials that react in an aggressive way with the environment, such as by producing an explosion or a rapid release of energy; and/or chemically-functionalized micro- and nano-particles which can react to some environmental parameter.

In accordance with an embodiment of the invention, a passive timing device such as the one previously described may be injected into a geological formation or in a hydraulic fracture by means of pumping via an injection well. When the timing mechanism triggers the piercing of the isolation membrane, functionalized nanoparticles are released within the geological formation as described above. The nanoparticles react with the local environment, are carried by flow towards the injection well, and are retrieved from the well at the surface. The nanoparticle size may be chosen to be substantially smaller than the average pore throat diameter, which will insure that the particle will be transported by flow within the geological formation without clogging the pores. By analyzing the particles after retrieval at the surface, one will be able to infer information about the environment within the geological formation at the time of nanoparticle release. By injecting multiple such passive timing devices which are triggered at different times, one may be able to continuously monitor one or several parameters at multiple remote locations within the geological formation, which may be otherwise inaccessible.

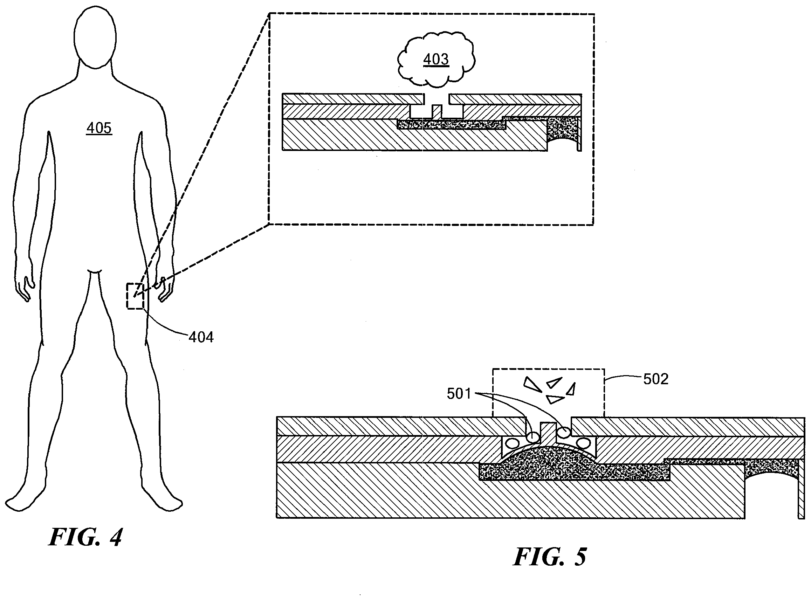

Fig. 4 shows a passive timing device **404** that includes, without limitation, a pharmaceutical product **403** that is released within a human body **405**, in accordance with an embodiment of the invention. The isolation diaphragm(s) is pierced at times set by the passive timing device, whereupon the corresponding pharmaceutical products **403** positioned, without limitation, within the isolated cavity and/or sampling chamber, are released within the human body. Multiple devices with one or more diaphragms may be utilized. Using such a system, complete treatment plans may be delivered without any active intervention, by adjusting the timing parameters and the types and quantities of pharmaceutical products within each cavity.

The device **404** may be attached to the skin of the human body **405**, or may be implanted within the body. An external source of pressure, or an external pump, may be used to drive the timing fluid within the timing cavity of the device **404**. In one embodiment, such external source of pressure may be, without limitation, a pressurized gas cartridge.

Fig. 5 shows a passive timing device that includes a filter **502** for containing the broken diaphragm particles, in accordance with an embodiment of the invention. Upon piercing of the mechanical structure (e.g., isolation diaphragm), the filter **502** advantageously prevents the broken diaphragm particles from passing into the external fluid, while still allowing, for example, a pharmaceutical product **501** to freely pass through. This embodiment may be particularly important if the passive timing device is going to be included within a human body.

### Tool implementation

The above-described devices may also be integrated within downhole sampling and measurements tools, such as the Modular Formation Dynamics Tester (MDT) produced by Schlumberger, the Formation Multi-Tester (FMT) produced by Baker Hughes or the Sequential Formation Tester (SQT) produced by Halliburton, or any other similar tool. Arrays of the sampling devices, integrating a plurality of devices and/or sampling mechanisms on a single microfabricated substrate, may be incorporated within the tool architecture. The above-described devices may also be integrated in production logging oilfield tools, possibly in slickline tools.

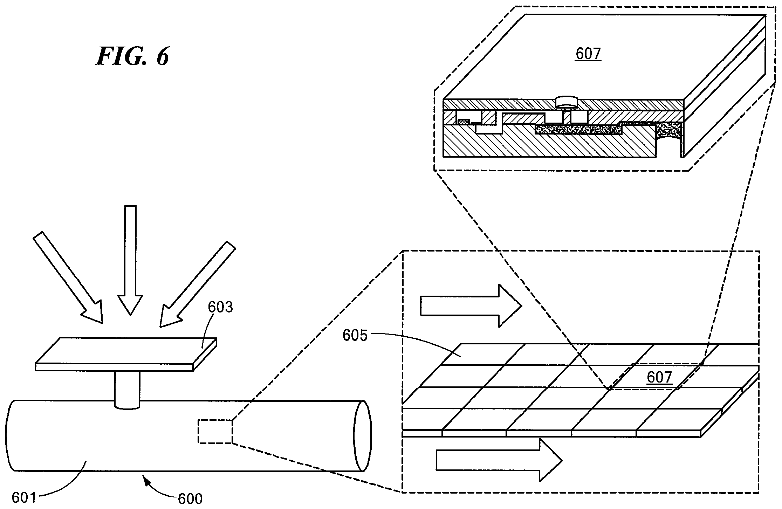

Fig. 6 shows integration of a plurality of sampling devices and/or mechanisms **605** within an oilfield-sampling tool such as a MDT, a FMT or a SFT, in accordance with an embodiment of the invention. The tool **600** pushes a pad **603** into the geological formation wall, and pumps the formation fluid into an internal flow-line **601**, where the fluid comes into contact with a smart sampling device array **605**. Each device **607** may acquire a sample, perform a measurement, and/or emit an acoustic signal which is recorded by a microphone within the tool. The recorded acoustic signals may provide, for example, the precise time when each measurement was performed and may uniquely identify the device which performed the measurement.

The device **607** may come into contact with the formation fluid as it is pumped into the tool flowline **601**. The acoustic emission events may be recorded using a microphone implemented in the tool, and later analyzed at the surface to infer the precise time of sample acquisition for each of the smart vessels in the array, thus providing very valuable time-series data.

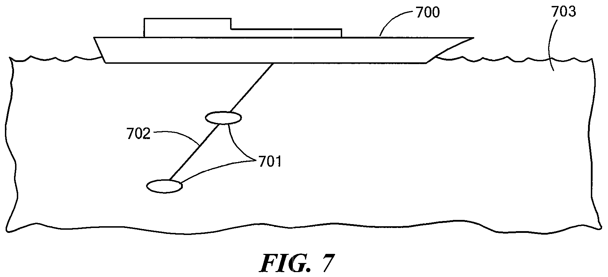

Fig. 7 shows another embodiment of the invention, where an array of smart sampling devices is embedded within a submarine measurement system **701**, which may be attached with a cable **702** to, without limitation, either a buoy, a rig, a submarine, a vessel or a ship **700**. The measurement system **701** may either be positioned in a stationary manner in the body of water **703**, at a depth dependent, without limitation, on the length of the cable **702**, or it may be dragged through the body of water by the ship **700**. The smart sampling devices in the measurement system **701** perform sample acquisitions and measurements at times determined by their respective timing mechanisms, thus providing a time-series or a spatial map of measurements at a given depth.

### Viscosity measurement

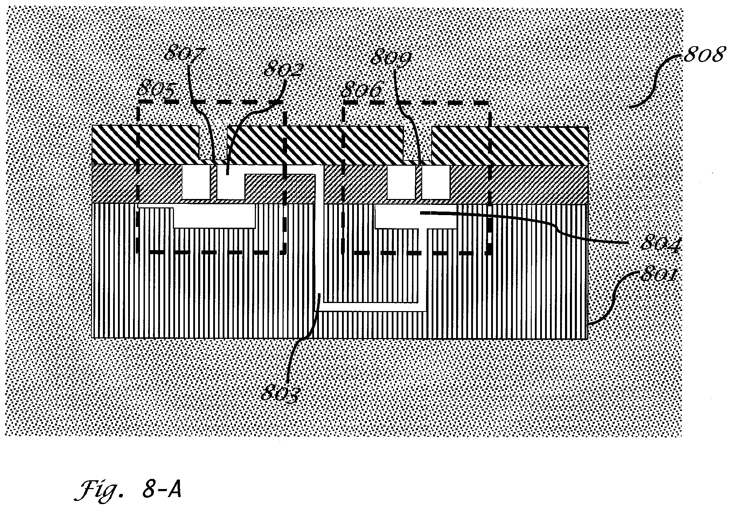

Fig. 8A shows a viscosity measurement system that may be fully passive, in accordance with an embodiment of the invention. The system **801** illustratively includes two devices **805** and **806** connected together in series, in such a way that the first device's isolated cavity **802** is connected, via a conduit, such as a microfluidic channel or other type of tube of controlled dimension **803**, to the timing cavity **804** of the second device. After the rupture or collapse of the first device's mechanical structure **807**, the second device's timing cavity **804** will start filling with external fluid **808**. The filling time of the second device's timing cavity **804**, and the corresponding rupture or collapse of the second device's mechanical structure **809**, may depend, at least in part, on the geometry of the conduit **803**, on external pressure, and/or on the viscosity of the external fluid **808**. By controlling, for example, the geometrical parameters, and by measuring external pressure, one can relate the viscosity of the external fluid to the time measured between the rupture or collapse of the first sampling device's mechanical structure **807** and that of the second device's mechanical structure **809**. This allows an accurate viscosity measurement to be performed on the external fluid.

More particularly, providing that the conduit **803** has hydrodynamic resistance *Rh,* the external fluid has pressure P, and the timing cavity **804** has volume *V*, the filling time *t* of cavity **804** will be given by *t=Rh×V*/*P.* The hydrodynamic resistance of a circular channel of radius R and length L is given by *Rh=8×n×L*/*(π×R⁴).* The hydrodynamic resistance, for a rectangular conduit of lateral dimension *h*<w and length L, can be approximated as *Rh=12×n×L*/*(h³×w×(1-0.63×hlw)),* where *n* is the viscosity of the external fluid (see, for example, D. Angelescu "Highly Integrated Microfluidics Design", Artech House 2011). By measuring the filling time of the cavity **804**, therefore, one can infer the value of the hydrodynamic resistance of the conduit **803**, and knowledge of the geometrical details of this conduit allows a determination of the fluid viscosity *n* from the above formulas: *n=t×P×π×R⁴*/*(8×L×V)* for a circular conduit, and, respectively, *n=t×P×h³×w×(1-0.63×h*/*w)*/*(12×L×V)* for a rectangular conduit.

In accordance with further related embodiments, the viscosity-measurement device described in the above paragraph may incorporate means of controlling and/or measuring the external fluid pressure, and of recording the time between the collapse of the first device's and the second device's mechanical structures **807** and **809**. The collapse of a device's mechanical structure may be detected acoustically (by detecting the acoustic signature emitted during the collapse), electrically (by recording a disruption to an electrical circuit caused by the collapse), or optically (by observing the collapse using a camera, or another type of optical system), or by any other means known to a person skilled in the art.

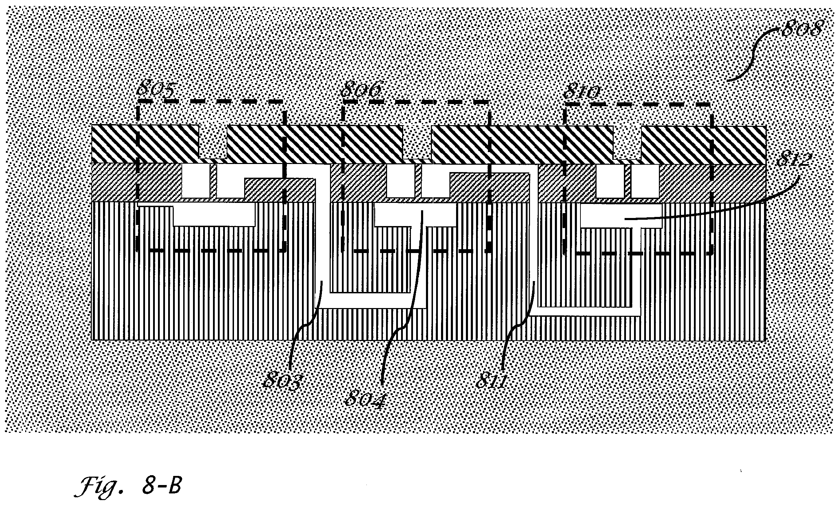

Fig. 8B shows another embodiment of the viscosity-measurement device incorporating an additional device **810** connected in series with devices **805** and **806** in such a way that the isolated cavity of device **806** is connected to the timing cavity of the device **810** by a second conduit **811** of controlled geometry. This second conduit **811** may have different cross-section and length from the first conduit **803**, thus resulting in a different timing fluid flowrate into the timing cavity **812** of the device **810**.

This difference in geometry between conduits **803** and **811** may be used to extend the measurement range of a device and measure different ranges of fluid viscosity using the viscosity-measurement device. In one embodiment, the geometry of conduit **811** may be chosen so that the filling time of cavity **812** is much longer than the filling time of cavity **804** (in case we assume equal volumes for the timing cavities **804** and **812**, this corresponds to the conduit **811** having significantly higher hydrodynamic resistance than conduit **803**). If viscosity of the external fluid is very low, and the filling time of the cavity **804** is too short to enable an accurate measurement, then a much more accurate measurement of viscosity may be obtained by using the filling time of cavity **811**. On the other hand, for highly viscous fluids, the filling time of cavity **804** may provide a reasonably accurate measurement, such that waiting for the filling of cavity **811** may no longer be necessary. Additional devices may be connected in series, with conduits connecting the isolated cavity of one device to the timing cavity of the next, to further extend the range of accurate viscosity measurements.

### Manifold sampling and chemical / biochemical measurement device

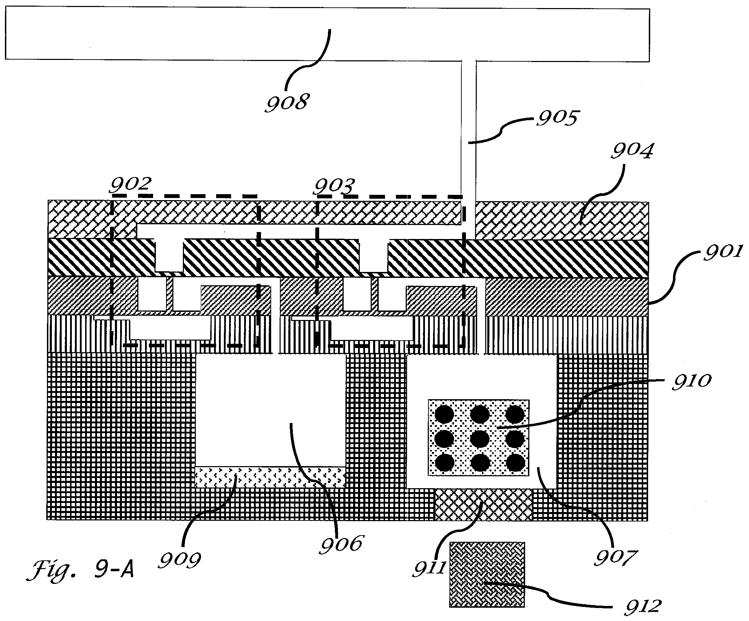

Fig. 9A shows a sampling and measurement system **901** that includes multiple devices **902**, **903** connected, via a manifold **904**, to an external sampling conduit **905**, in accordance with an embodiment of the invention. Upon a new sample being acquired by one of the devices, new fluid may be drawn through the sampling conduit **905**. The sampling chambers **906**, **907** for the devices **902**, **903** may be designed with volumes such that the dead volumes within the system, the internal volume of the conduit **905** or the volume of the connecting manifold **904** be negligible in comparison to the volume of the sampling chambers **906**, **907.**

The sampling conduit **905** may be connected to a pipe, a fluid reservoir, or another external fluid supply **908** that needs monitoring. Each time a new sample is acquired by one of the devices **902**, **903**, the respective volume of fluid is drawn from the said fluid supply **908**, through the conduit **905** and manifold **904**, into the sampling chamber of the active device.

Each sampling chamber **906**, **907** may be, without limitation, a vial, a bottle and/or another leakproof container/receptacle. One or more sampling chambers **906, 907** may include a pre-measured amount of chemical or biological reagent **909**, or a combination of several such reagents **910** in liquid, solid, powder or lyophilized form, in free form or immobilized on a solid substrate. Upon sample entering the sampling chamber **906, 907,** a sequence of chemical or biological reactions occur between the sample and the said reagents. Different sampling chambers **906**, **907** within the same system may contain different reagents **909**, **910**.

In various embodiments of the invention, said chemical or biological reactions may have a visible outcome. For example, the coloration of the solution or of an immobilized reagent may change, there may be a change in turbidity, there may be a development of fluorescence, or any combination of the above.

The visible outcome may be recorded in-situ, by performing an optical measurement via, without limitation, the vial wall or via an optical window **911** embedded in the sampling chamber **907**. The optical measurement may include, without limitation, acquiring an image of the sampling chamber using an external optical instrument **912** such as color or black and white camera, a spectrophotometer, a fluorescence detection device, a Raman scattering device, and/or a turbidity measurement device.

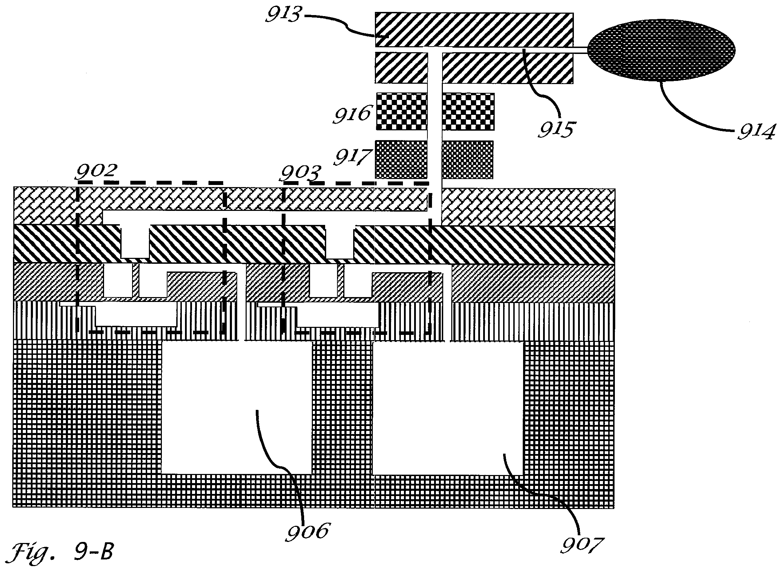

Fig. 9B shows an external sampling conduit connected, via a T-junction **913**, to a reagent reservoir **914**, in accordance with an embodiment of the invention. The reagent reservoir **914** may be substantially at the same pressure as the fluid being sampled. For example, and without limitation, the reagent reservoir **914** may be a bladder submerged into the external fluid being sampled, or an accumulator. A fluidic resistance **915** may be included between the reagent reservoir **914** and the T-junction **913**, such as to limit the flow rate of reagent. Each time a sample is drawn in, a proportional amount of reagent is drawn in along with the sample, the mixing ratio being set passively by the said fluidic resistance.

The external sampling conduit may include a micromixer **916** downstream from the T-junction **906**, **907**, such that the combined sample and reagent stream is thoroughly mixed after passing through the micromixer **916**.

In various embodiments of the invention, the external sampling conduit may include a micro fluidic sensor **917**, such that each time a sample is acquired by one of the devices, the microfluidic sensor **917** performs a measurement on the fresh stream of fluid. The measurement may include, without limitation, an optical measurement (e.g., index of refraction, absorbance, fluorescence), an electrical measurement (e.g., conductivity, resistivity, dielectric constant), an electrochemical measurement (e.g., ionic content, chemical composition), a physical measurement (e.g., viscosity, density), a chemical measurement (chemical composition), and/or biological measurement (cell count).

### Preconcentration and/or sample filtering

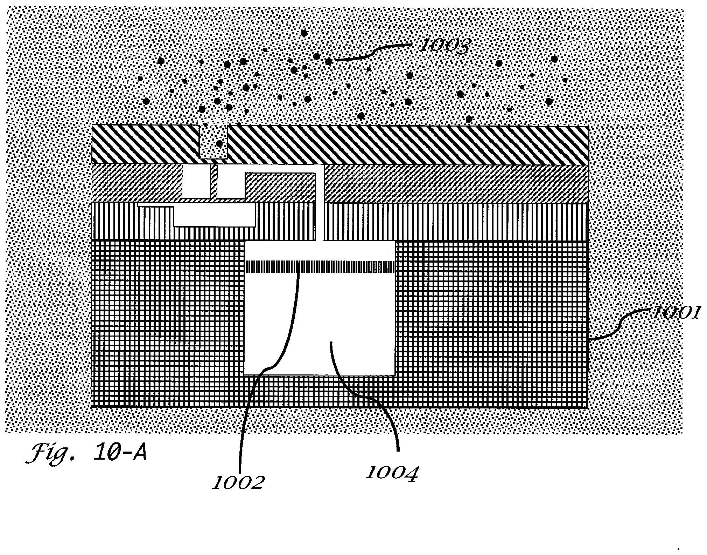

Fig. 10A shows a device **1001** that may be used to sample from an external fluid contaminated with one or a combination of particles **1003**, organic pollutants, biological pollutants, chemical pollutants, plankton, phytoplankton, nuclear matter, volatile organic compounds, pharmaceutical matter, or other contaminants, in accordance with an embodiment of the invention. The device **1001** includes one or more integrated filters **1002** that the sample has to come in contact with, upon or prior to entering the sampling chamber **1004**. The filter **1002** may include, without limitation, one or a combination of the following: a mechanical filter, a solid phase extraction column, a packed column, a hydrocarbon filter, a gas chromatography preconcentrator, a filter to collect and concentrate radioactive material, a biological filter, an absorbent medium, a scavenging medium, a hydrophobic material and a hydrophilic material.

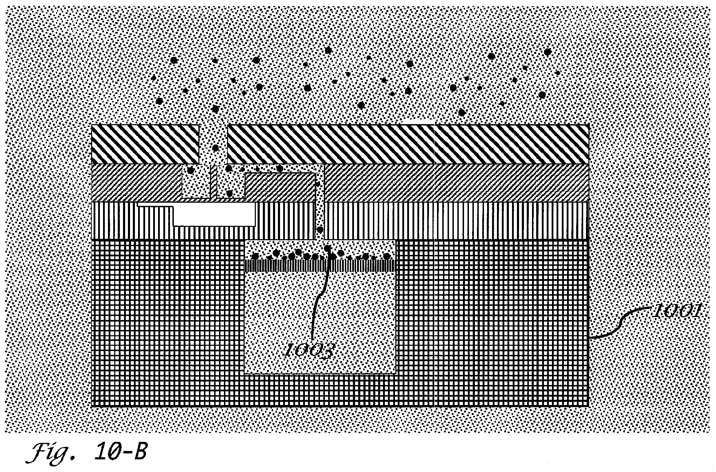

Fig. 10B shows the device **1001** after sampling, the filter **1002** having collected different components present in the fluid sample, such as, without limitation: particles **1003**, organic pollutants, biological pollutants, chemical pollutants, plankton, phytoplankton, nuclear matter, volatile organic compounds, pharmaceutical matter, or other contaminants.

The filter **1002** may, optionally, be later retrieved and analyzed, to provide time-series data concerning the contaminant of interest at the location of the device. Analyzing the filter **1002** may require backflushing, thermal desorption or solvent washing, and/or other techniques to remove the adsorbed, absorbed, or trapped contaminants. Analysis may require analytical techniques such as, without limitation, GC/MS, HPLC, gamma ray spectroscopy. In various embodiments, the filters may be analyzed in-situ.

### Maintaining Sample Integrity by Controlled Sampling and High-Pressure Preservation

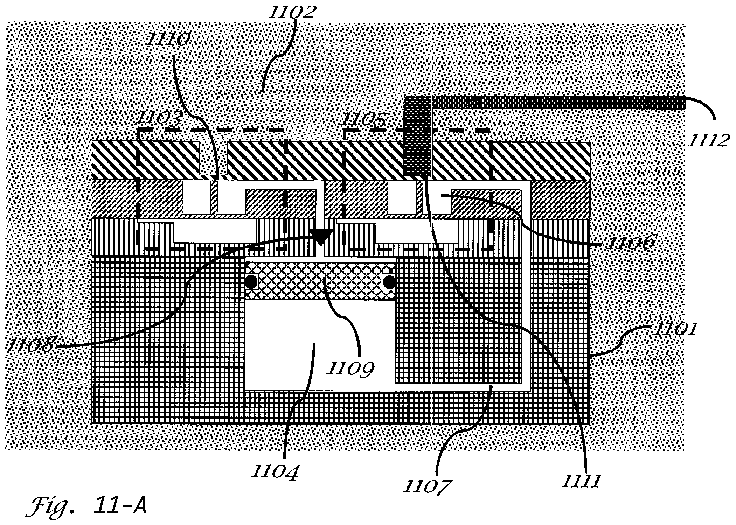

Figs. 11A-C shows a system **1101** capable of acquiring a sample from an external fluid **1102**, pressurizing it at a pressure higher than the pressure of the external fluid, and maintaining it at such pressure for extended periods of time, in accordance with an embodiment of the invention. Such a system **1101** advantageously may ensure that the sample will remain in single-phase configuration and will not undergo a thermodynamic transition to a multi-phase fluid. This is particularly important when sampling hydrocarbon fluid from a geological formation during oilfield drilling, wireline logging, or production operations, since the sample needs to remain in single phase throughout the transport to the analysis laboratory.

The system **1101** shown in Fig. 11A includes a sampling chamber **1104** divided into a first portion and a second portion, in accordance with an embodiment of the invention. The isolated cavity of a first device **1103** is connected via a conduit to the first portion of the sampling chamber **1104**. The second portion of the sampling chamber **1104** is connected via a conduit **1107** to an isolated cavity **1106** of a second device **1105**. The mechanical structure **1111** of the second device **1105** may be, without limitation, scheduled to open at a later time relative to the mechanical structure **1110** of the first device **1103**. The first portion of the sampling chamber **1104** may be separated from the external fluid by a first check valve **1108**, allowing fluid to enter the sampling chamber **1104** but not to leave it. The connection between the two portions of the sample chamber **1104** may include a second check valve allowing flow from the second portion of the sampling chamber **1104** to the first portion of the sampling chamber **1104,** but preventing flow in the opposite direction. The first and second portions of the sampling chamber **1104** may be separated by, without limitation, a leakproof piston **1109** or a flexible membrane, allowing for the pressures in the first and second portions to be equalized without requiring physical contact of the fluids in the two portions. The mechanical structure **1110** of the first device **1103** may be connected to the fluid to be sampled **1102**, with the mechanical structure **1111** of the second device **1105** connected to a pressurized fluid reservoir **1112** at a pressure that is higher than the external pressure. The pressurized fluid reservoir **1112** may incorporate, without limitation, an accumulator, a pressurized gas container, and/or a mechanical spring.

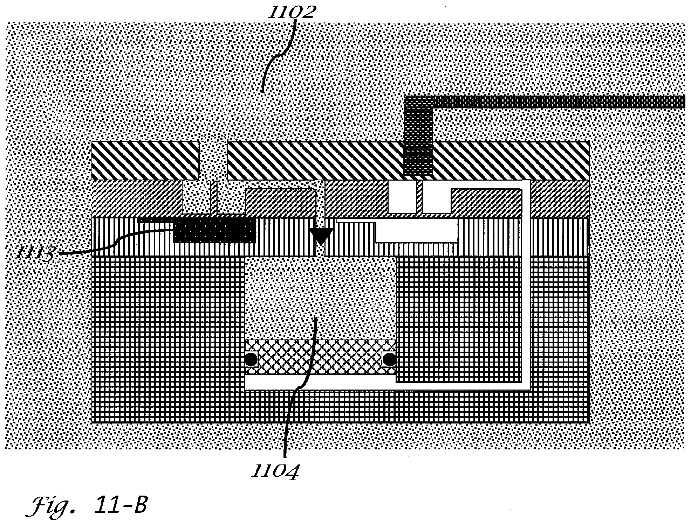

Fig. 11B shows the system **1101** with the timing fluid cavity **1113** of the first device **1103** filled with timing fluid, causing the rupture or collapse of the first device's mechanical structure **1110,** and allowing the external fluid **1102** to enter the first portion of the sampling chamber **1104.**

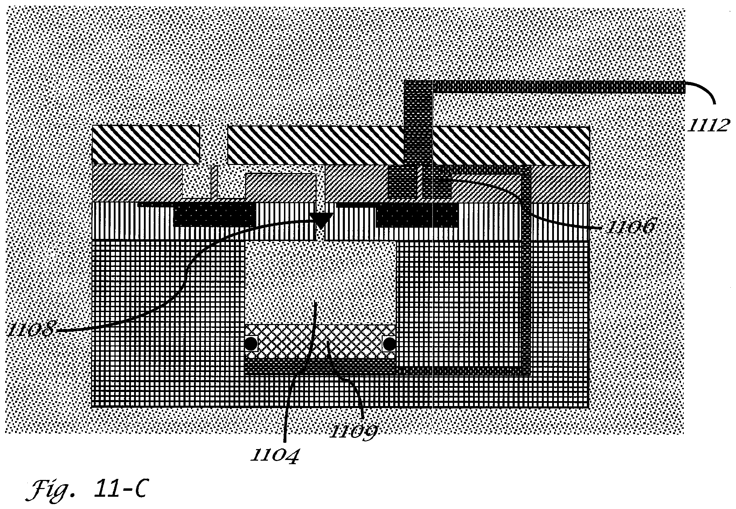

Fig. 11C shows the subsequent rupture or collapse of the second device's mechanical structure **1114**, the fluid from the pressurized reservoir **1112** entering the second portion of the sampling chamber **1106** and applying its pressure, via the piston **1109**, to the sample contained in the first portion of the sampling chamber **1104.** The fluid in the first portion of the sampling chamber **1106** is prevented from leaving the chamber by the check valve **1108**, and therefore is maintained at a pressure higher than the pressure at which it was acquired.

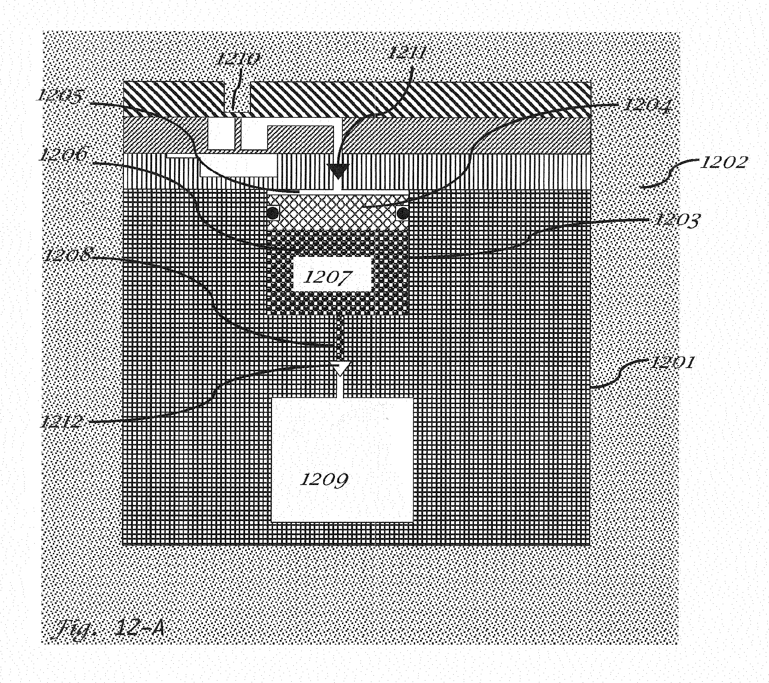

Fig. 12A shows a device **1201** for performing a sampling operation from a high-pressure external fluid **1202** without significantly lowering the pressure of the external fluid during the sampling process (without "shocking" the fluid), in accordance with an embodiment of the invention. The sampling chamber **1203** may include a leak-proof piston **1204** or flexible membrane that separates it into a first portion **1205** and a second portion **1206.** The second portion **1206,** which is farther away from the mechanical structure **1210** may be pre-filled with a secondary liquid **1207** and is connected, via a conduit **1208**, such as a microfluidic channel and/or fluidic constriction, to another auxiliary chamber **1209.** The conduit **1208** may include a check valve **1212** or backpressure regulator that assures that the fluid **1207** does not leak from the second portion **1206** to chamber **1209** prior to sample acquisition. Upon the collapse of the mechanical structure **1210,** the sample enters the first portion **1205** of sampling chamber **1203** and applies pressure to the piston **1204,** which moves at a slow rate controlled, for example, by the viscosity of the secondary liquid **1207** and the geometry of the conduit **1208.**

In another related embodiment, the auxiliary chamber **1209** may be pre-filled with pressurized gas. Upon sample acquisition, the gas is compressed, forming a cushion that will keep the secondary liquid **1207,** and consequently the sample, pressurized. The sample in the first portion **1205** of the sampling chamber **1203** is prevented from leaving by the check valve **1211,** and therefore is maintained at a pressure that is comparable to the pressure at which it was acquired.

In another related embodiment, the sampling chamber may include a compressed spring and a piston, one side of the piston in contact with the sampling chamber and the other side in contact with the external fluid, such that prior to sampling being initiated the spring is compressed by the piston due to external fluid pressure being applied to the piston. Upon sampling being initiated, the hydrostatic pressure on both sides of the piston equalizes and the elastic force of the spring displaces the piston, thus acquiring a sample at controlled speed and with minimal change to the overall submerged weight and buoyancy of the device. The travel of the piston may be restricted due to the presence of a mechanical fixture such as a stop or a ridge.

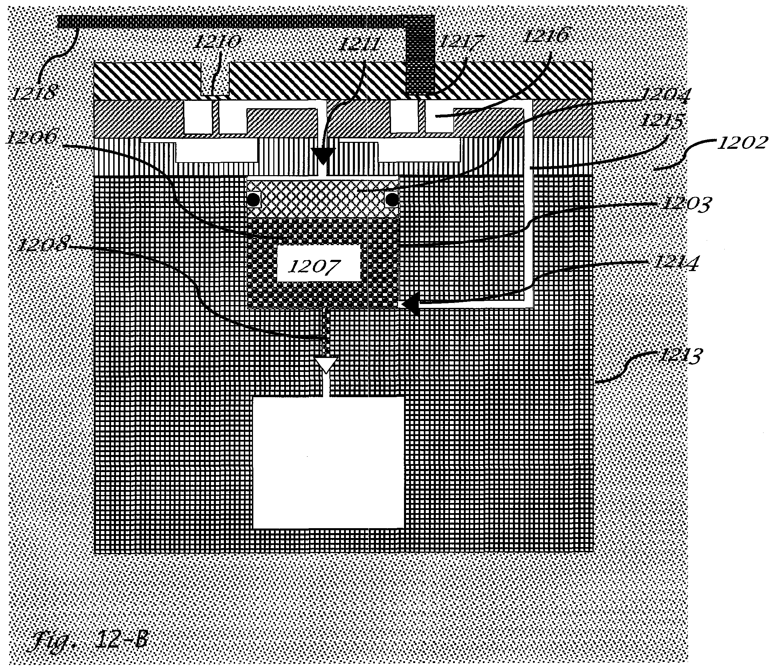

Fig. 12B displays another embodiment of Fig. 12A that in addition to allowing a sampling operation to be performed from a high-pressure fluid without dropping the pressure of the fluid or otherwise "shocking" it during the sampling, also allows the sample to be maintained at a high pressure after sampling, possibly higher than the initial external fluid pressure, thus preventing phase separation. In addition to the details provided above with regard to Fig. 12A (the numbering of which will be maintained), in the system **1213** the second portion **1206** of the sampling chamber **1203** that is farther away from the mechanical structure **1210** is also connected via a conduit **1215** to an isolated cavity **1216** of a second device, which is scheduled to open at a later time. The conduit **1215** may include a check valve **1214** allowing flow from the isolated cavity **1216** to the second portion **1206** of the sampling chamber **1203,** but preventing flow in the opposite direction. Positioned between the first portion **1205** and the second portion **1206** of the sampling chamber **1203** may be a piston or a flexible membrane allowing for the pressures to be equalized without requiring physical contact of the fluids in the two portions **1205** and **1206.** The mechanical structure **1210** of the first device is connected to the fluid to be sampled **1202**, whereas the mechanical structure **1217** of the second device is connected to a pressurized fluid reservoir **1218** at a pressure that is higher than the external pressure. The pressurized fluid reservoir **1218** may incorporate, without limitation, an accumulator, a pressurized gas container, and/or a mechanical spring. Upon the rupture or collapse of the first device's mechanical structure **1210**, external fluid **1202** is allowed to enter the first portion **1205** of the sampling chamber **1203**, at a slow rate that is controlled, at least in part, by the viscosity of the secondary liquid **1207** and/or the geometry of the conduit **1208**. Upon the subsequent rupture or collapse of the second device's mechanical structure **1217,** the fluid from the pressurized reservoir **1218** enters the second portion **1206** of the sampling chamber, and applies its pressure to the piston **1204,** and consequently to the sample contained in the first portion **1205** of the sampling chamber **1203**. The fluid in the first portion **1205** of the sampling chamber **1203** is prevented from leaving the check valve **1211**, and therefore is maintained at a pressure higher than the pressure at which it was acquired.

### Complex Sample Manipulations, Filter Backflushing, and Transfer Between Vials

Figs. 13A-D show, in chronological order, operations performed by a system that, in addition to sampling at a time controlled by a passive timing mechanism, integrates a mechanism allowing the subsequent transfer of the sample from an initial sampling chamber to another sampling chamber after a given amount of time, in accordance with an embodiment of the invention. This operation can be further repeated as desired. The system described in Figs. 13A-D may also integrate different chemical or biochemical reagents in each of the sampling chambers, and/or may integrate a filtration medium that can be backflushed as part of the sample transfer to another sampling chamber, thus concentrating certain components of the sample.

FIG 13A shows a system **1306** that includes multiple sampling devices **1301**, **1302**, **1303**, such that a sample from external fluid **1304** may be acquired by a first device **1301** and provided to a first portion of a sampling chamber **1305**. The first portion of sampling chamber **1305** may optionally include a filter **1307**. The first portion of the sampling chamber **1305** may also integrate an optional first group of chemical or biochemical reagents. The first portion of the sampling chamber **1305** may be separated from a second portion of the sampling chamber **1305** by a piston, or a flexible member membrane. The second portion of the sampling chamber **1305** is in fluidic communication with an isolated cavity **1308** of a second device **1302** that is scheduled to open at a later time. The first portion of the sampling chamber **1305** is also connected, upstream of the optional filter **1307**, to the mechanical structure **1312** of a third device **1303** that is scheduled to open after the second device **1302**. The mechanical structure **1313** of the second device **1302** is in turn connected to a pressurized fluid reservoir **1314**, such as, without limitation, a pressurized gas reservoir, or an accumulator.

Fig. 13B shows the system **1306** after the collapse of the first device's mechanical structure **1309**, showing a sample **1310** being acquired into the first portion of the sample chamber **1305**, mixing with the optional first reagent of group of reagents, and pushing the piston **1311** into a far position distal the first device's mechanical structure **1309**.

Fig. 13C shows the system **1306** after the later collapse of the second device's **1302** mechanical structure **1313**. The pressurized fluid from reservoir **1314** enters the second device **1302** and applies its pressure to the backside of the piston **1311**. The sample acquired in the first portion of the sampling chamber **1305** cannot backflow into the first device **1301** due to the check valve **1315**.

Fig. 13D shows the system **1306** after the later collapse of the third mechanical structure **1312**. The sample **1310** contained in the first portion of the sampling chamber **1305** is pushed, by the pressure of the pressurized gas reservoir **1314**, into the sampling chamber **1315** of the third device **1303**. During this process, the sample is forced to traverse the optional filter **1307** in reverse, thus back-flushing it and transporting the filtered material into the sampling chamber **1315** of the third device **1303**. By choosing the volumes of the sample chamber **1315** of the third device **1303** to be lower than the volume of the first portion of sample chamber **1305**, a higher concentration of the components filtered from sample **1310** can be obtained in the sampling chamber **1315**. The sample chamber **1315** of the third device **1303** may also include an optional second group of chemical or biochemical reagents, such that the sample **1310**, after having already reacted with the optional first group of reagents present in the first portion of the sample chamber **1305**, now has to react with the optional second group of reagents. The process may be repeated multiple times.

Fig. 13E shows the above-described system **1306** slightly modified, with the sampling chamber **1305** further including a first reservoir **1316** that may be pre-filled with a liquid solution **1317**, and a second reservoir **1318** that initially may be empty. The first reservoir **1316** may be separated using a piston **1319** or flexible membrane, or a similar structure, from the second portion of the sampling chamber **1305** that is in fluidic communication with the isolated cavity **1308** of the second sampling device **1302**. Optionally, one-way check valves **1320**, **1321** may be integrated allowing the fluid to circulate from the filter **1307** into the second reservoir **1318**, and, respectively, from the first reservoir **1316** towards the filter **1307**. Upon the sample acquisition by the first device **1301**, the sample moves through the filter **1307** into the second reservoir **1318**. Upon the collapse of the mechanical structure **1313** of the second device **1302**, pressure from pressurized reservoir **1314** is applied to the liquid solution **1317** in the first reservoir **1316**. As the mechanical structure **1312** of the third device **1303** collapses, the liquid solution **1317** is forced through the filter **1307**, and transports the material collected on filter **1307** into the sampling chamber **1315** of the third device **1303**. Optionally, a first and a second group of reagents may be incorporated in the first portion of the sampling chamber **1305**, and, respectively, in the sampling chamber **1315** of the third device **1303**. Additional reagents may be included in the liquid solution **1317** present in the first reservoir **1316**.

The operation mode described above allows complex sample preparation, such as, without limitation, mixing with multiple chemical or biochemical reagents, backflushing using a specific liquid solution that is different from the original sample liquid, preconcentration in a separate vial, and additional chemical and/or biochemical reactions on the preconcentrated sample.

### Daisy chain configuration of multiple sampling systems

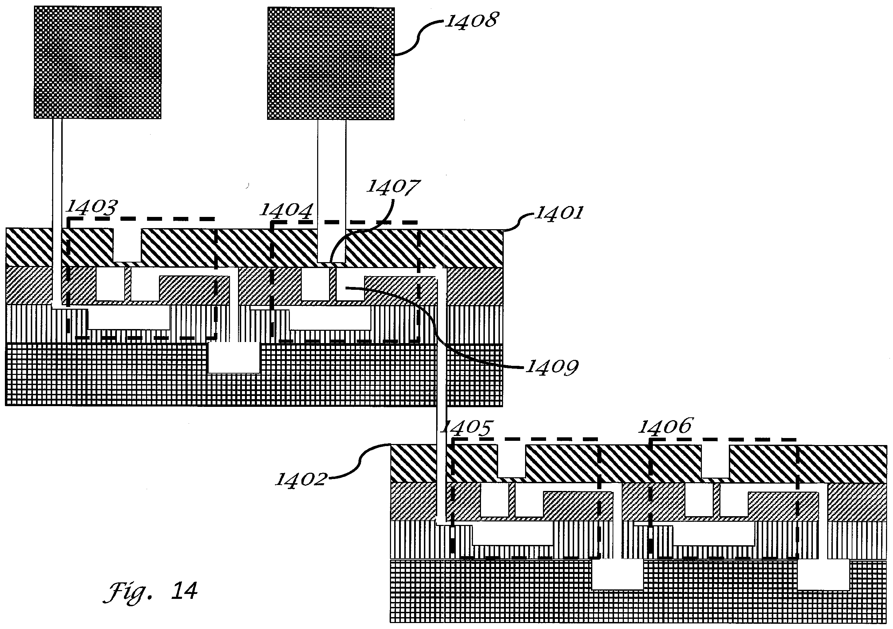

Fig. 14 shows multiple systems connected in a "Daisy-chain" configuration, in accordance with an embodiment of the invention. Upon one system acquiring its last sample it automatically triggers the start of sampling using the next system in the daisy chain. This mode of operation can allow an unlimited number of systems to be connected, and thus extends the sample acquisition capacity of the combined system beyond the limits of any single individual system connected in the daisy chain.

Two systems **1401** and **1402**, each including a plurality of devices **1403** and **1404**, and **1405** and **1406**, respectively, are timed to acquire corresponding samples at different times. Illustratively, the mechanical structure **1407** of the last device **1404** of the first system **1401** may be connected to the timing fluid reservoir **1408** of the second system **1402**. Additionally, the isolated cavity **1409** of the last device **1404** of the first system **1401** may be connected to the timing mechanism of one or more of the devices **1405**, **1406** associated with the second system **1402**. In this configuration, the collapse or rupture of the mechanical structure **1407** of the last sampling device **1404** of the first system **1401** triggers the start of the sampling using the second system **1402**, thus allowing the systems to be connected in a daisy-chain configuration.

### External control of the sampling time

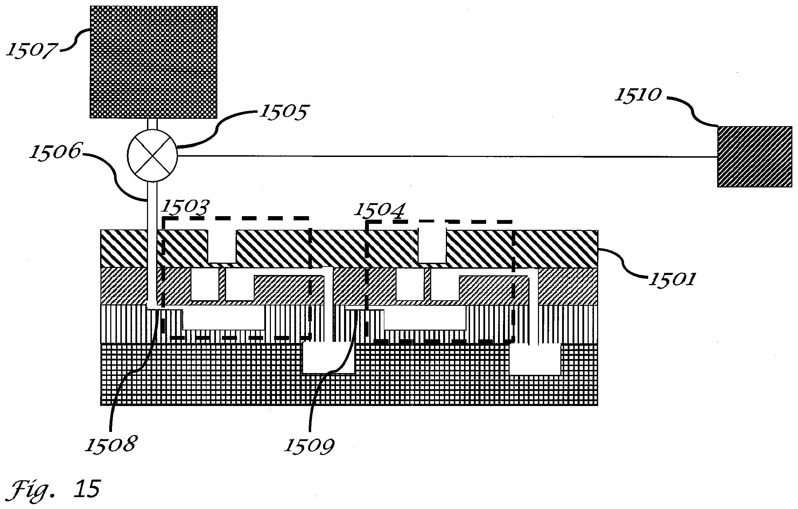

FIG 15 shows a system **1501** capable of controlling the sampling times of a plurality of sampling devices **1503** and **1504** using a trigger device **1505** that may be in the form of a fluid control device. The trigger device **1505** may be placed, without limitation, on a timing fluid line **1506**, between an optional pressurized timing fluid reservoir **1507** and the timing channels **1508**, **1509** of any number of the multiple timing devices **1503**, **1504** associated with the system **1501**. The trigger device may be controlled by a control unit 1510 that may either be a subsystem of system **1501**, or an external, possibly remote, system.

The pressurized timing fluid reservoir **1507** may be absent, instead the timing fluid may be maintained at a pressure equal to the external fluid being sampled. The trigger device **1505** can be any type of device that can enable or disable the passage of timing fluid as desired. The trigger device **1505** may be one of a check valve, an electrically-controlled solenoid valve, a fluidic switch, or any other type of active valve known in the art. The trigger device **1505** may also include a one-shot valve, that initially blocks the passage of timing fluid, and upon receipt of an external signal from the control unit **1510** permanently opens the passage of timing fluid without requiring further power.

In accordance with further embodiments, the system **1501** may incorporate a recording mechanism that records that a sample has been acquired, and/or of transmitting this information to either an external system, to the control unit **1510,** or both. The collapse or rupture of the mechanical structure and the subsequent sample acquisition may be detected acoustically (by detecting the acoustic signature emitted during the collapse), electrically (by recording a disruption to an electrical circuit caused by the collapse), optically (by observing the collapse using a camera, or another type of optical system, or by observing an optical change to a vial being filled with fluid), or by any other means known in the art.

### Passive timing and sample acquisition implemented using a piston assembly

In certain applications, using a timing diaphragm inside a sampling mechanism may not be convenient or ideal. Instead it may be advantageous to use a different type of moving part that is capable of achieving a good fluidic seal.

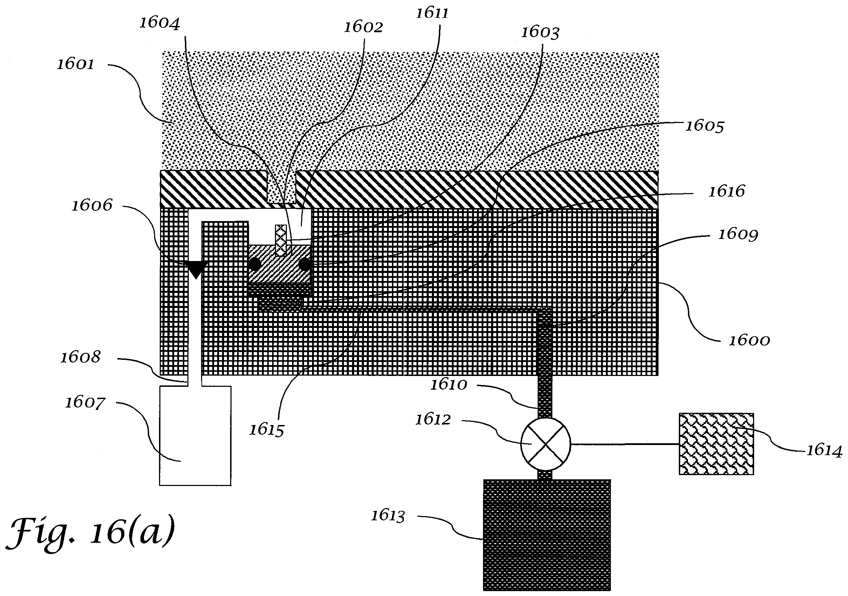

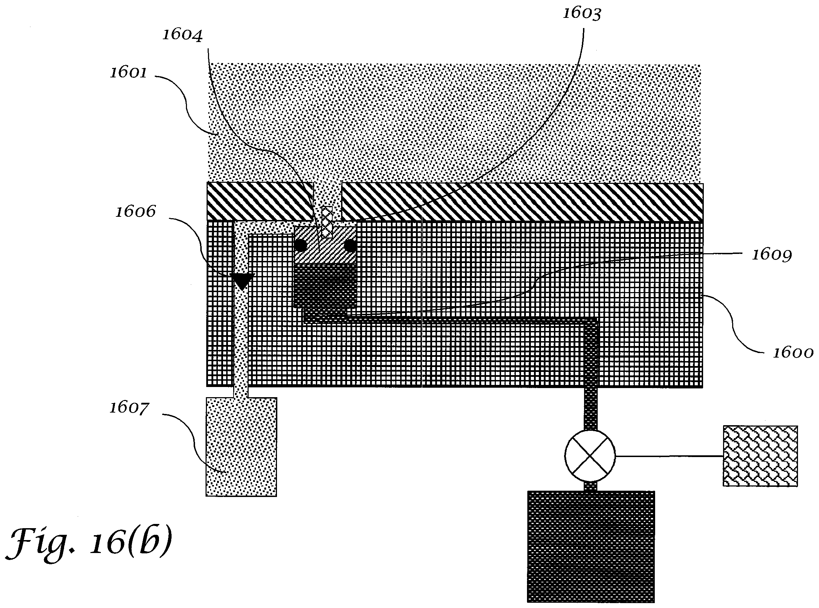

Figs. 16A and 16B show a sampling device **1600** that is configured to acquire a sample from an external fluid **1601**, in accordance with an embodiment of the invention. The sampling device **1600** includes a mechanical structure **1602** that is in contact with the external fluid **1601**. The sampling device **1600** further includes an isolated cavity **1611**. A piston **1604** is positioned, without limitation, within the isolated cavity **1611**, and is configured to move within said cavity **1611**. In various embodiments, the piston **1604** separates said cavity **1611** into two portions that are not in fluid communications because of a sliding seal **1605**. The sliding seal **1605** may be, for example, an o-ring or any other type of seal known in the art that performs a sealing function while allowing the piston **1604** to slide. The sampling device **1600** may further include a timing cavity **1616** in fluid communication with one side of the piston.

The sampling mechanism may further include a conduit **1615** that may be, without limitation, a microfluidic channel or a capillary tube, and that may have a predefined geometry. Upon applying pressure to the timing fluid **1609** (which may be a liquid or a gas), said timing fluid **1609** flows within the conduit **1615** at a rate, for example, that may be dictated by the applied pressure, the predefined channel geometry and known timing fluid properties. Upon reaching the timing cavity **1611** and filling it after a timing interval, the timing fluid **1609** applies pressure to one side of the piston **1604,** which advances within the isolated cavity **1611** (alternatively called a piston cavity).

The piston **1604** may also include a piercing structure, such as a protrusion **1603,** which may be, without limitation, in the form of a needle, a pin, a raised boss, or any other type of structure known in the art. The protrusion **1603** may be separate from the piston **1604** or an integral part of it. Upon the piston **1604** sliding far enough into the isolated cavity **1611**, the protrusion **1603** contacts the mechanical structure **1602** and transmits and/or concentrates mechanical stress onto the mechanical structure **1602**.

Under the effect of said stress, the mechanical structure **1602** is pierced and the mechanical structure **1602** is destroyed, such as by, without limitation, rupturing or by collapsing, allowing the external fluid **1601** to enter the isolated cavity **1611,** which may then further lead to a sampling chamber **1607**. The isolated cavity **1611** and the sampling chamber **1607** may be part of the same assembly as the sampling mechanisms, or they may be separate parts that are connected using some form of fluidic or mechanical fixture known to the person skilled in the art, such as a tube, a channel, or a pipe **1608**. Prior to entering the sampling chamber **1607**, the external fluid **1601** may pass through a check valve **1606** that allows fluid to flow into the sampling chamber **1607** but prevents the fluid **1601** from flowing in the opposite direction.

The timing fluid **1609** may further be in fluidic communication via, without limitation, a tube **1610,** a channel, or a pipe, or any other type of fixture or device, that allows fluidic communication with a pressurized timing fluid reservoir **1613.** In some embodiments, the timing fluid reservoir **1613** may be at a pressure that is equalized with the pressure of the external fluid **1601.** In other embodiments, the timing fluid **1609** may be the same fluid as the external fluid **1601**. The tube **1610** may be optionally connected to an on/off valve **1612**, which may be manually operated or controlled by an optional control device **1614**.

The control device **1614** and the valve **1612** may be electrically active. The control device **1614** may be triggered remotely. The triggering action in itself may be transmitted to said control device **1614** via a mechanical, acoustical, electrical or electromagnetic wired or wireless link. For example, the triggering action may be transmitted to the control device **1614** using, without limitation, a mechanical cable or lever, a serial communication cable, a parallel communication cable, an electrical triggering cable, an electromagnetic wave using a mobile telephony network or a radio frequency or satellite connection, a pressure wave such as an acoustic or sound wave using an acoustic module (such as sonar and/or a hydrophone, a speaker and a microphone, or similar), or any other form of acoustic, electrical, electromagnetic, acoustic or mechanical communication and/or triggers known in the art.

Fig. 16B shows the sampling device **1600**, after the piston **1604** has moved under the effect of pressure from the timing fluid **1609**, and the protrusion **1603** of the piston **1604** has pierced the mechanical structure **1602**. Sample chamber **1607** is shown filled with external fluid **1601**.

### Triggered sampling implementation with different passive timing durations

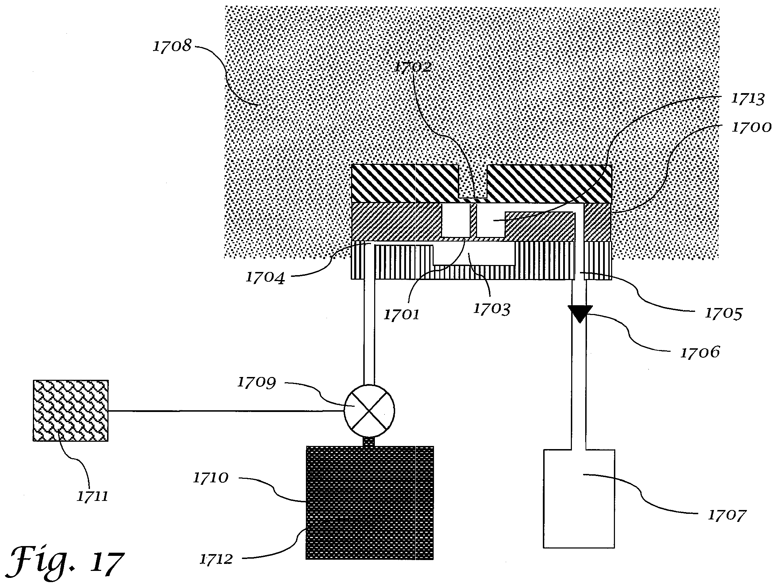

Fig. 17 shows a configuration of a sampling device **1700** that allows said sampling device **1700** to be in a stand-by mode for an extended period of time, and then, upon receiving an external trigger, to initiate an electrically passive timing operation and to acquire a sample from external fluid **1708** after said electrically passive timing operation is performed, in accordance with an embodiment of the invention. The device **1700** includes a timing diaphragm **1701**, and a mechanical structure **1702** initially in contact with the external fluid **1708** and separating it from an isolated cavity **1713**. The mechanical structure **1702** may be pierced and consequently destroyed (such as by collapsing or rupturing) by the action of the timing diaphragm **1701**. The device may further include a conduit **1704**, such as, without limitation, a channel, a tube, a microfluidic channel, or a pipe, and a timing cavity **1703**.

The conduit **1704** is in fluidic communication with a pressurized timing fluid reservoir **1710**, and may optionally include an on/off valve **1709**. The valve **1709** may be actuated manually, or remotely by an optional control device **1711**. An external trigger may act on the control device **1711**, which in turn activates (turns on) the valve **1709**. Once the valve **1709** is turned on, the timing fluid **1712** starts advancing within the conduit **1704** and, after a time interval, fills the timing cavity **1703**. The pressure of the timing fluid **1704** is applied to the timing diaphragm **1701**, which acts on the mechanical structure **1702** and destroys it, thus allowing a sample of the external fluid **1708** to fill the isolated cavity **1713**.

The isolated cavity **1713** may optionally be connected to a sampling chamber **1707** via, without limitation, a tube **1705**, channel, or pipe or any other type of fixture or device known in the art that allows fluidic communication. The tube **1705** may optionally include a check valve **1705**, that allows fluid to flow into the sampling chamber **1707** but prevents it from flowing in the opposite direction.

The control device **1711** and the valve **1709** may be electrically active. The control device **1711** may be triggered remotely. The triggering action in itself may be transmitted to said control device **1711** via, without limitation, a mechanical, acoustical, electrical or electromagnetic wired or wireless link. For example, the triggering action may be transmitted to said control device **1711** via a mechanical cable or lever, a serial communication cable, a parallel communication cable, an electrical triggering cable, an electromagnetic wave using a mobile telephony network or a radio frequency or satellite connection, a pressure wave such as an acoustic or sound wave using an acoustic module (such as sonar and/or a hydrophone, a speaker and a microphone, or similar), or any other form of acoustic, electrical, electromagnetic, acoustic or mechanical communication and/or triggers known to the person skilled in the art.

This embodiment allows the sampling device **1700** to acquire a sample after a time interval following an external trigger, said time interval being measured, starting from the external trigger, using an electrically-passive timing mechanism. The sample acquisition operation, including in this case by the piercing and destruction of the said mechanical structure by the timing diaphragm **1701**, is also based on purely hydraulic and mechanical action, and therefore is electrically passive.

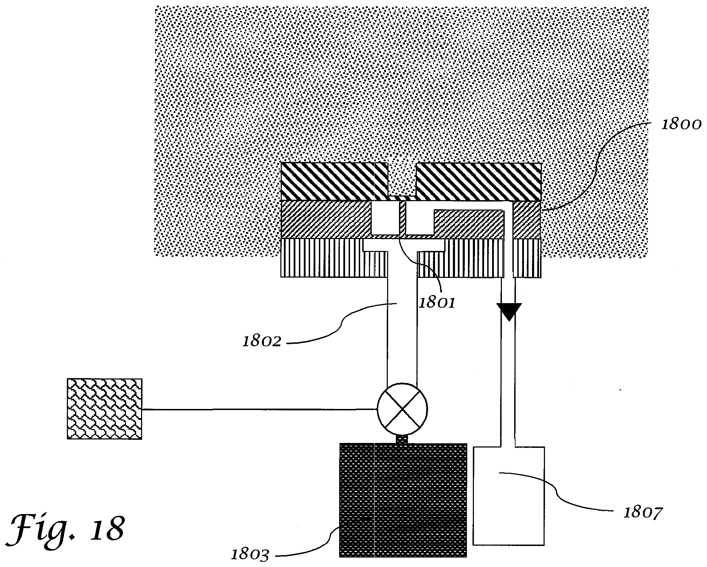

Fig. 18 shows a sampling device **1800,** in accordance with an embodiment of the invention, that is similar to the above-described device **1700,** as shown in Figure 17, but having a modified conduit **1802** for the timing fluid **1803**, and/or a reduced timing cavity volume **1801**, thus leading to a decrease of the said timing interval separating an external trigger from the sampling acquisition operation. The timing interval may be further reduced by using a timing fluid **1803** having lower viscosity than timing fluid **1712**. Timing fluid **1803** may be a gas. The timing interval may thus be reduced substantially, in the range of, without limitation, 0.1ms to 10ms for certain applications where rapid response to an external trigger is required. In other applications, the time interval may be, illustratively, less than 100 ms, or range from 10ms to 1s. In other applications, the time interval may range from 1s to 100s. In other time applications the required timing may be significantly longer.

### Modifying sampling timing by changing the timing cavity volume

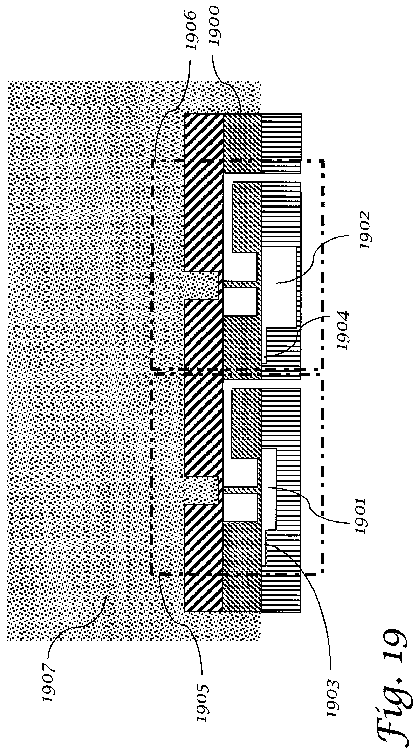

Fig. 19 shows another sampling device configuration, in accordance with an embodiment of the invention. The sampling device **1900**, configured to acquire one or several samples from an external fluid **1907**, includes at least two electrically passive sampling mechanisms **1905** and **1906**. The two or more sampling mechanisms **1905** and **1906** are designed to acquire their respective samples at different times, and differ at least in the fact that the volume of the timing cavity **1902** of one of the sampling mechanisms **1906** is larger than the volume of the timing cavity **1901** of another sampling mechanism **1905**. The corresponding timing fluid conduits **1904** and **1903** may be identical, or they may differ in geometry. The two or more sampling mechanisms **1905** and **1906** may be within a single sampling device as shown, or various system embodiments may include multiple sampling devices having different timing cavity volumes.

### Ocean pollution monitoring system deployment

In illustrative embodiment of the invention, a system is provided that may be put in place around, without limitation, an industrial facility as a precautionary measure at an early stage in the project. The systems may then be activated remotely for deployment of the sampling arrays, for example, in the event of a serious failure situation, even if control at the facility has been completely lost. The sampling arrays may be used, without limitation, to detect the scope of leaks or other pollution resulting from the failure situation. Additional fail safes may be implemented which allow for the activation of the devices in several modes on remote command from surface via an acoustic or other type of transmission. Such a system may be deployed as a fully contained unit that sits on the seafloor or other body of water in standby mode, and allows for normal operations at the facility to continue unimpeded in absence of any alert or accident.

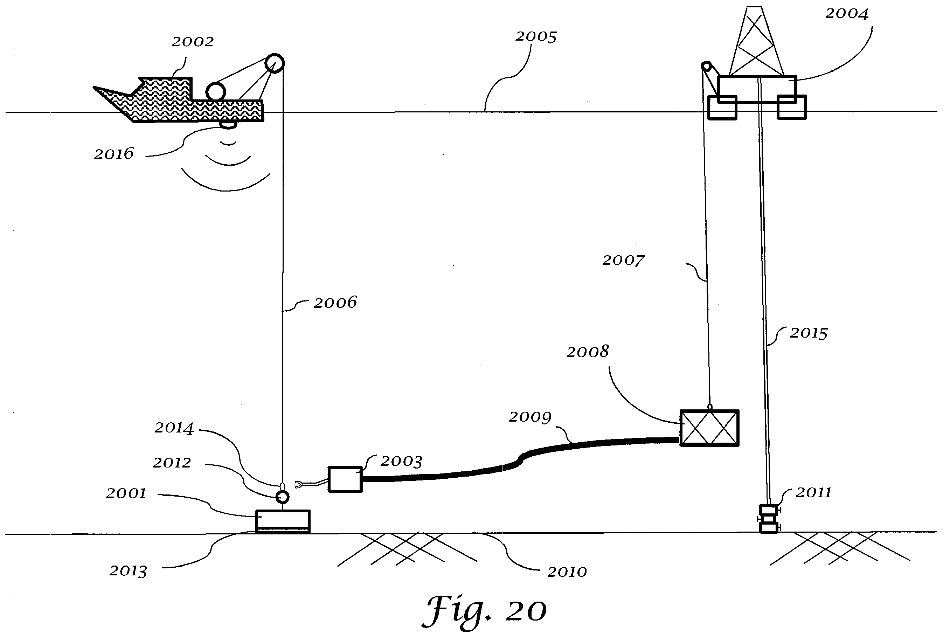

More particularly, FIG. 20 shows an installation of passive sampling systems **2001** around, without limitation, oil rigs, offshore platforms or other industrial facility **2004** that may be, for example, located in a large body of water such as the ocean, in accordance with an embodiment of the invention. The sampling systems **2001** may be located, without limitation, in a fully self contained unit, which may be installed by a surface vessel or ship **2002** that may or may not have the assistance of an ROV (Remotely Operated Vehicle) **2003**. The sampling systems **2001** may be placed in an accurately known geographical location on the sea floor **2010** relative to the wellhead **2011** or as determined by GPS (Global Positioning System) coordinates, or both. The sampling system **2001** may be installed via an installation / retrieval tether **2006** by the surface vessel. This tether **2006** serves to lower the sampling system **2001** in a controlled manner from sea level **2005** to the sea floor. The self contained unit may have sufficient weight to keep the entire system negatively buoyant. In various embodiments, an anchor element 2013 may be integrated into the self contained element, or otherwise attached to the self contained unit. The sampling system **2001** may include a buoyancy device **2012** which serves as a deployment vehicle for a sampling array (described in more detail below) once activated. The system **2001** also may include a latch / release mechanism **2014**, which allows for the disengagement of the installation tether **2006** either remotely by the surface vessel **2002**, or physically by the ROV **2003**. The latch/release mechanism **2014** may also act as a latch point for future retrieval. The installation / retrieval tether **2006** may also act as a way to keep the system **2001** suspended at neutral weight during installation such that the ROV **2003** would be able to move it in the horizontal direction, and accurately position the sampling system on the sea floor. The final position of the sampling system **2001** on the sea floor may be determined by the ROV sonar with relative position to the wellhead, and riser **2015**. In addition, surface sensors located on the surface vessel **2002** such as downward facing sonar **2016**, or similar technology used to locate objects below the ocean surface may be used to determine the final position of the sampling system **2001**. Additionally emitters, reflectors or other devices may be positioned on the sampling system **2001** or installation tether **2006** to aid in the surface detection of the subsea position of the system **2001** during the system installation. The ROV **2003** may or may not be needed for the assistance, guidance or monitoring of the system installation, location and/or detachment of the installation tether **2006**. The ROV **2003** can be deployed from either the surface facility **2004** or the surface vessel **2002** and may be deployed directly from surface or via an ROV cage tether **2007**, ROV cage **2008** and ROV tether **2009**.

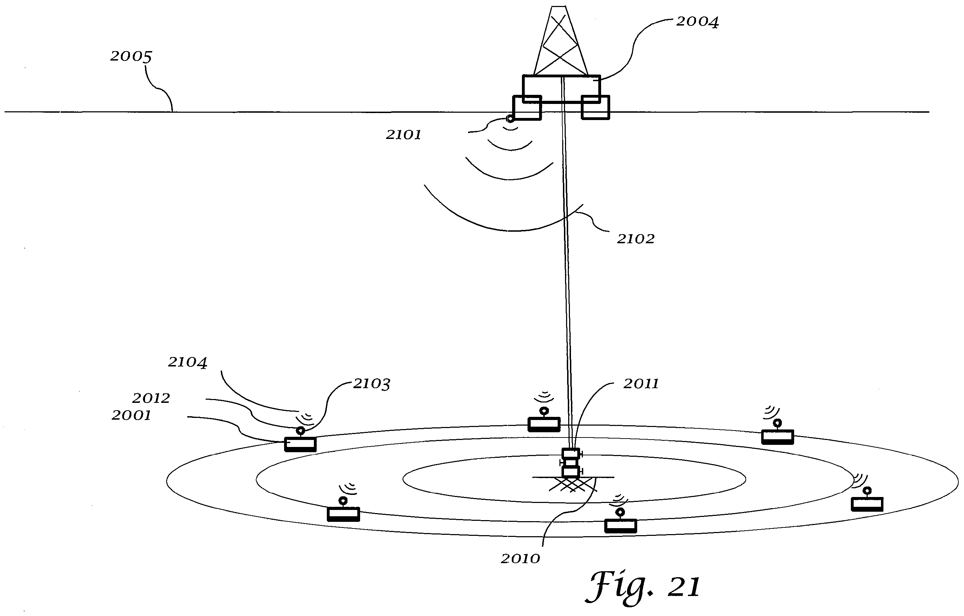

Fig. 21 shows multiple sampling systems **2001** installed in an array on the sea floor around an offshore surface facility **2004**, in accordance with an embodiment of the invention. The sampling systems **2001** may be arranged in any manner, layout or number without limitation. Located, without limitation, on the surface facility **2004** may be a surface transmitter / receiver **2101** which is able to transmit a control signal **2102** through the water column. The signal **2102** may be, without limitation, an acoustic or optical signal, or any other type of signal known to one of ordinary skill in the art. The transmitted acoustic signal may be projected in all directions and is not limited to only vertical or line-of-sight transmission. Each sampling system **2001** may include a subsea receiver / transmitter **2103** which has the ability to receive the signal **2104**. On receipt of the signal **2104** from the surface facility **2004**, the subsea receiver **2103** may either remain in standby mode, or move to sleep mode, or act as a trigger for further action within the system, depending on the signal received.

In various embodiments, each system **2001** may have the ability to transmit an acknowledgement signal, or small packet of data back to the surface facility **2004**. The sampling systems **2001** may remain in standby mode as long as a transmission command is received to do so from the surface facility **2004**. The system **2001** may be programmed to wake up at predefined timed intervals and listen for a control signal, such as, without limitation, an acoustic signal or an optical signal, from the surface facility **2004**. If the system **2001** receives the transmission from the surface facility **2004**, it is an indication of regular operation from the surface facility **2004**, and the system **2001** can go into sleep mode for a specified period of time in order to conserve power. After the specified period of time, the system **2001** will again wake up and check for the transmission from the surface facility **2004**. This may continue indefinitely and keep the systems **2001** in standby mode for the period of installation which may be days, weeks or many months in duration, as long as normal operations continue. In addition, an extra surface transmitter may be installed as a backup system to avoid unintended activation and deployment.

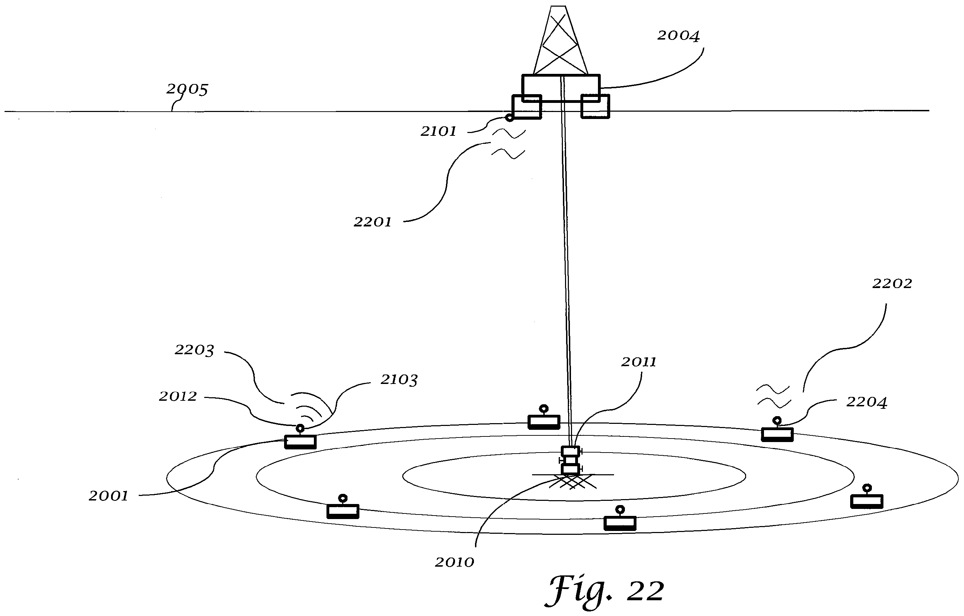

Fig. 22 shows an event occurring at the surface facility **2004** and either power is cut to the surface transmitter **2101** (illustratively shown as an acoustic transmitter), or the transmission has been intentionally stopped by someone on the surface facility (i.e. control room personnel), in accordance with an embodiment of the invention. In this case, there is a loss of transmitted signal **2201** and thus a loss of received acoustic signal **2202** at the subsea acoustic receiver **2103**. The protocol within the sampling system **2001** may change at this point, and remain awake, continuing to listen for the acoustic transmission from the surface facility **2004**. At this point the subsea acoustic transmitter **2103** may emit a transmission signal or alert **2203** to the surface acoustic or optical receiver **2101** as warning to the surface facility **2004** that no surface transmission has been received. The sampling systems **2001** will remain awake in delay activation mode for a period of time which may be on the order of minutes or hours. If after this period of time, still no acoustic signal is received from the subsea acoustic transmitter **2103**, a release latch mechanism **2204** may be activated by a trigger mechanism for release of the sample array (described in more detail below).

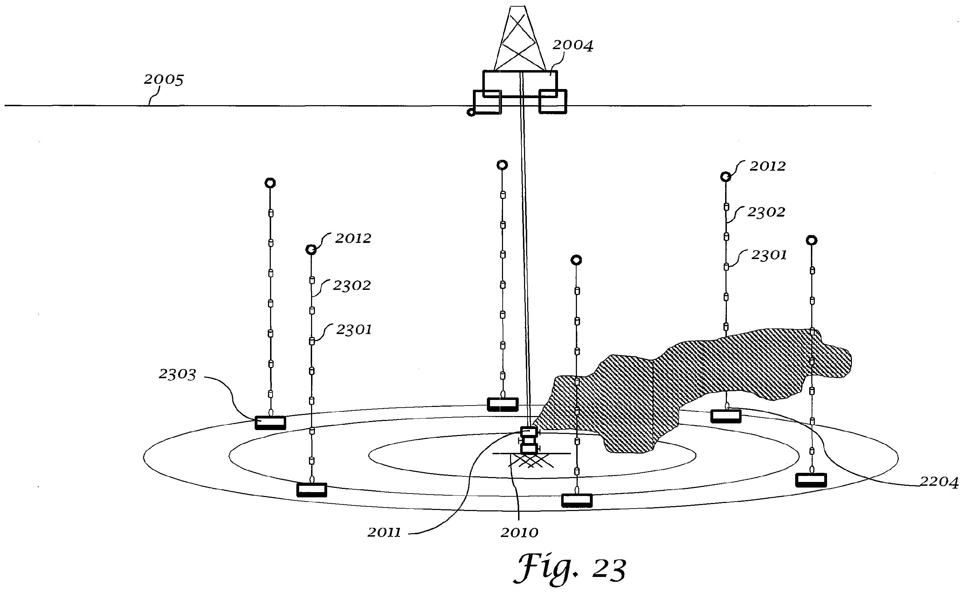

FIG. 23 shows the result of deployment of a sampling array **2302** by release of the release latch mechanism **2204**. Each sampling array **2302** may include any number of sampling devices **2301** attached to, without limitation, a rope or cable, in any spacing configuration, and may extend from sea floor to sea level, or any point in between. Upon activation of the buoyancy device **2012** to disconnect at the release latch **2204**, the buoyancy device **2012** will ascend through the water column. The buoyancy device **2012** may be attached by a sampling cable to each of the sampling devices **2301** in series, such that during the ascent, each sampling device **2301** is pulled out of the containment unit **2303** and into sampling position. Alternatively, each sampling device **2301** may itself be buoyant in which case an additional buoyancy device **2012** may or may not be used. The vertical spacing of each sampling device **2301** may be determined by the length of cable that is installed between each sampling device **2301**. The entire sampling string may be tethered at one end to the containment unit **2303** and anchor element **2013** to keep the entire sampling array in position. Each sampling device **2301** may be automatically triggered mechanically or electrically as it is pulled from its initial position relative to the containment unit **2303**, so as to start the timing fluid for time interval sampling. This trigger may be by Hall Effect sensor, or mechanical switch, or other device with similar functionality to allow for activation of the timing fluid. In various embodiments, each sampling device **2301** may be triggered by simply deploying/entering the body of fluid (for example, the body of fluid may be used as the timing fluid). Once activated, each sampling device **2301** may acquire separate individual samples at specified timed intervals that are preprogrammed prior to installation of the sampling systems **2001.**

The trigger mechanism may control the latching mechanism **2204** by providing to the latching mechanism **2204** an acoustic signal, an electric signal, an optical signal, an electromagnetic signal, or a mechanical signal, or a combination thereof. As described above, the containment unit may include a receiver for receiving a control signal that may be an acoustic signal or an optical signal, the trigger mechanism controlling the latching mechanism as a function of the control signal. The trigger mechanism may include an acoustic release, a device commonly used in fields such as oceanography. The latching mechanism **2204** may include a fusible wire and means of sending an electrical current through the fusible wire, leading to the melting of the fusible wire and the release of the sampling array. The latching mechanism **2204** may further include means to providing mechanical advantage to the strength of the fusible wire.

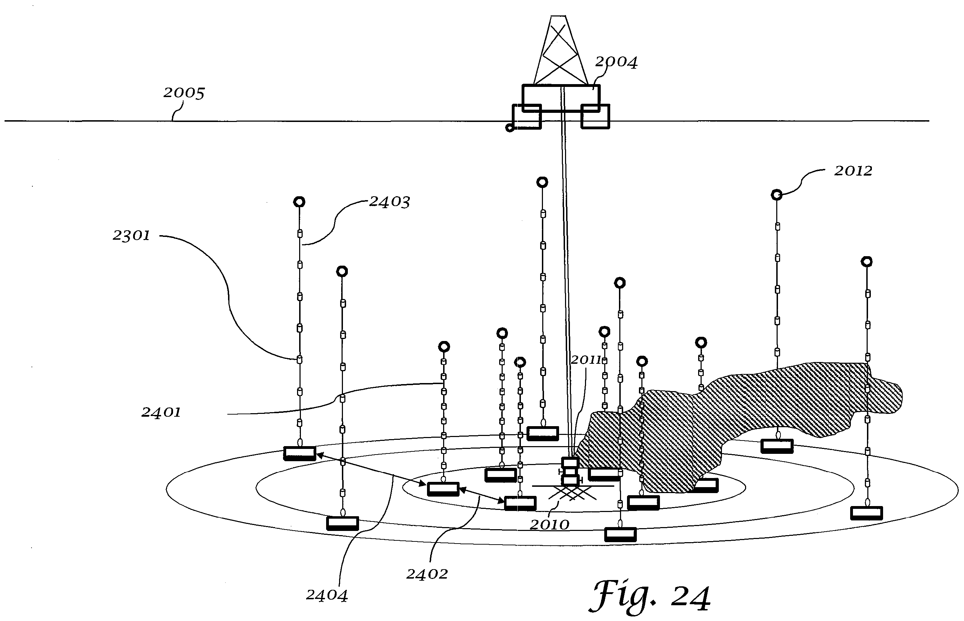

Fig. 24 shows deployment of monitoring systems that include near-well systems having a closer spacing both vertically **2401** as well as horizontally **2402**. This provides a greater resolution of the pollution profile in 3 dimensions at the critical areas near to the wellhead **2011** over time to give a more accurate map of the pollution progression. At a greater perimeter from the wellhead **2011**, the systems may have a larger spacing horizontally **2404**, extend further vertically in the water column and may have a greater spacing **2403** between each individual sampling device **2301** in the sampling array. These sampling arrays may include any number of sampling devices in each array depending upon various parameters such as ocean depth or distance from the wellhead **2011** or both. The sampling arrays may also be biased or clustered in a particular direction relative to the wellhead **2011** based on known variables such as ocean current direction or ocean depth profile or shore line direction or other such factors.

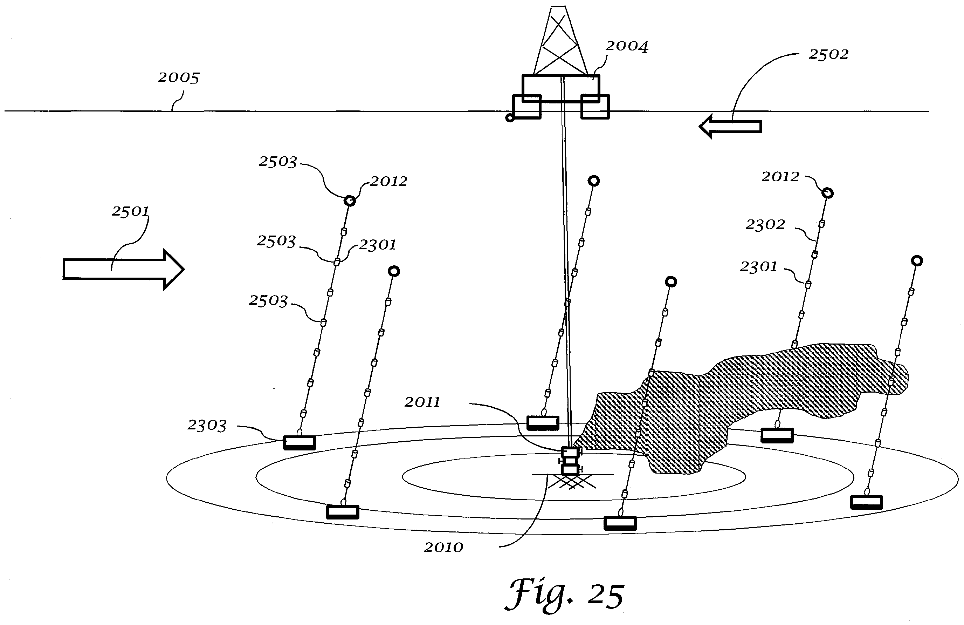

Fig. 25 shows sampling arrays **2302** deployed in a non-static environment, in accordance with an embodiment of the invention, such as the case when there is natural ocean current **2501** or wave motion **2502** due to winds, or other movement within the ocean column. This movement may or may not be in the same direction and may cause one or more sampling arrays **2302** to be in a position that is not vertically above the original known anchoring point. In illustrative embodiments, one or multiple devices or sensors may be installed into the sampling array that are capable of measuring the array's actual position. Using such devices, the position of each sampling device in a sampling array may be accurately determined over time relative to the known anchor point.

More particularly, a device **2503** may be installed in the sampling array that includes a 3-axis accelerometer or tilt meter or inclinometer or compass or relative bearing device or flow meter or any combination of such devices or similar, such that the position of each sampling device **2301** in the string may be accurately determined over time relative to the known anchor point of the self contained unit. The device **2503** may be contained in the buoyancy device **2012** at the top of the sampling array **2302**, or at a point near the top of the sampling array **2302**. Additionally, any multitude of such devices **2503** may be included at multiple points along the sampling array **2302** or within the sample devices **2301**. This configuration would allow for the prediction of each sampling device **2301** position in the case that forces within the ocean column are not uniform, such as variable ocean currents, resulting in a sampling array **2302** that may or may not be vertically linear. Additionally, the associated sampling devices **2301** and cable of the sampling array **2302** may be designed to be either neutrally buoyant or positively buoyant as to allow for greater confidence in the predicted position of each sampling device **2301**. The device(s) **2503** would record their continuous positioning data throughout the sample acquisition in order to provide positional data of each sample device **2301** at each sample acquisition time for input into prediction models. In addition, this continuous positioning, directionality and or ocean current and flow data may be used in itself for input into prediction models beyond the point by point positional data provided only at the time of each of the acquired samples.

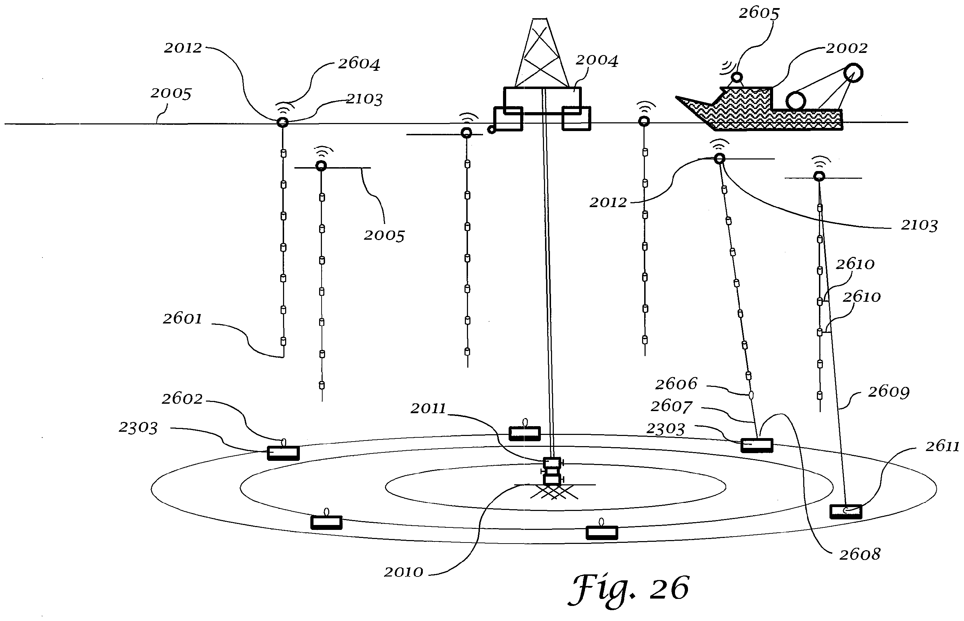

Fig. 26 shows several options for retrieval of the sampling arrays and/or sampling systems after the samples have been acquired, in accordance with an embodiment of the invention. In various embodiments, a sample array release **2601** may be activated and separated from a latch element **2602** at the completion of all sampling. The sampling array **2302** remains attached to the buoyancy device **2012** which then ascends to sea level **2005** at the ocean surface. Latch element **2602** may also act as a connector for future retrieval of the containment unit **2303**. At the same time, the retrieval transmitter **2103** located on or inside the buoyancy device **2012** may transmit a locator signal **2604**, which may be in the radio frequency range for detection and location by the retrieval vessel **2002**, which may carry a locator device **2605** to detect the location of these sampling arrays **2302**. In addition, the buoyancy device **2012** may be a color that is highly marine visible for easy visual detection on the ocean surface. The retrieval transmitter **2103** may also contain lights that will additionally aid in the visual detection and retrieval of the sampling strings, especially during but not limited to night operations. The retrieval vessel **2002** with the locator device **2605** may determine the location of the sampling arrays **2302** by radio signal strength or by signal directionality or by visual observation or any combination of these methods. In another case, at the completion of all sampling, a sampling array release **2606** is activated which separates the sampling array **2302** from the latching element **2602** located on the containment unit **2303,** however, the array **2302** is still attached to a retrieval tether **2607.** This retrieval tether **2607** allows the sampling array **2302** to travel to the ocean surface, but still maintains connection to the containment unit **2303** by a connection element **2608.** This connection element **2608** may be attached to a wire coil that is located within the containment unit body that is able to expel cable as the buoyance device **2012** ascends to surface. The buoyancy device **2012** may also be of a color that is highly marine visible and may contain a retrieval transmitter **2103** as described previously. The retrieval tether **2607** may be such that the length is enough to allow the buoyancy device **2012** to reach surface, or may be long enough that the tether **2607** itself can extend to surface for retrieval of the containment unit **2303** by a mechanical winch system. In this case the buoyancy device **2012** may act as a locator for the sampling array **2302**, and also provide a direct connection to the containment unit **2303** for subsequent retrieval.

In yet another embodiment, the buoyancy device **2012** may include a separate retrieval tether **2609** that is attached to the containment unit **2303**, but is able to spool **2611** itself out as the buoyancy device **2012** is released from the containment unit **2303** and ascends through the water column. This tether **2609** may be at a minimum, long enough to reach from sea floor **2010** to ocean surface **2005**. The retrieval tether **2609** may also be such that it deploys in multiple stages. One such example would be that the retrieval tether **2609** is programmed prior to installation to release from the containment unit **2303** when commanded to do so, and unspool a certain length of cable. This would effectively set the depth of each sampling device relative to the sea floor **2010** for the duration of the timed sampling. Then after a predetermined period of time, or at a given trigger, or at the end of sampling, the retrieval tether **2609** unspools until the buoyancy device **2012** reaches the ocean surface **2005**. In this case the sampling array **2302** or sampling devices **2301** may or may not be attached by an attachment line **2610** to the retrieval tether **2609**, but the retrieval tether **2909** remains fixed to the containment unit **2303** on the sea floor **2010**. The attachment line **2610** may be of any length, and may be used as an alternative to the cable/string of sampling array **2302** itself. Additionally, the spooling device **2611** may be placed in different points within the sampling system **2001**, including mounting on or contained within the buoyancy device **2012** itself.

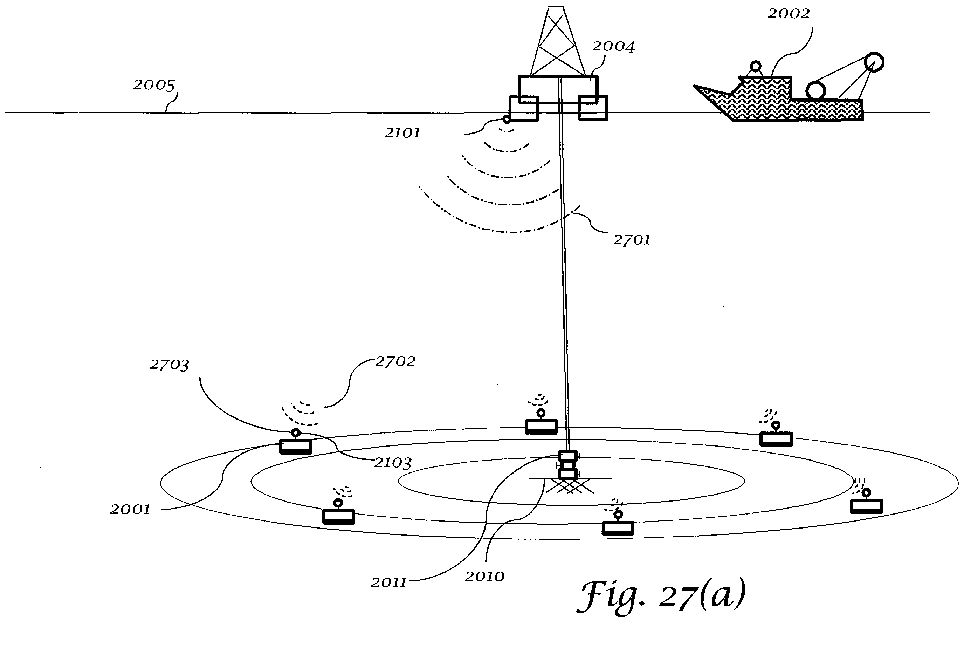

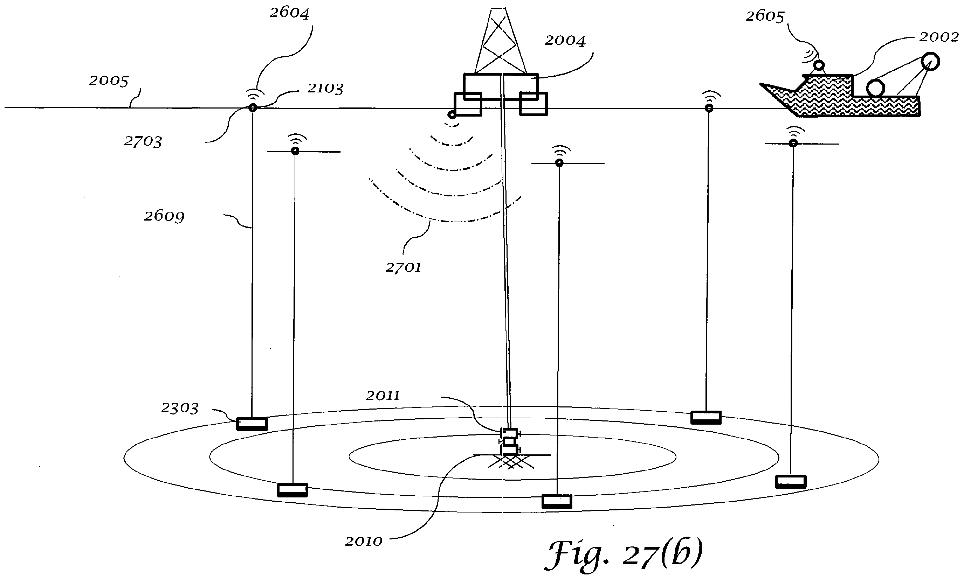

Figs. 27(a) and 27(b) show the activation of a transmitted acoustic signal that acts as an override for sampling system retrieval, in accordance with an embodiment of the invention. The surface acoustic transmitter **2101** is capable of transmitting an override acoustic signal **2701** to the sampling systems that are in standby mode on the ocean floor. When an override acoustic signal **2702** is received at the subsea acoustic receiver **2103**, the system acts to deploy a buoyancy device **2703** that pulls a retrieval tether **2709** fully to the surface. This may be performed without activation of the sampling array **2302** or sampling devices **2301**. Each unactivated system may be retrieved from the sea floor for subsequent deployment at a later time, or at a different location, or both. In various embodiments, the system may contain a safety protocol, such that activation of the system cannot be triggered while the tether is physically connected to the buoyancy device or other connection point. This may be performed by Hall Effect sensor or mechanical safety lock or other method to achieve similar function. The system may be intended for repeated deployment with routine servicing and maintenance. The buoyancy device **2703** may be the same device as the previously described buoyancy device **2012**, or may be a secondary buoyancy device. The buoyancy device **2703** may have an additional function whereby the override acoustic signal **2702** is able to select either to deploy the sampling array **2302**, or to deploy the retrieval tether to the surface, or a combination of either, using a single buoyancy device. The buoyancy device **2012** and buoyancy device **2703** may be combined together in series and deployed independently or may be deployed simultaneously to provide an easy method for sampling and immediate retrieval after sampling. The buoyancy device **2703** may also be of a color that is highly marine visible and may contain a retrieval transmitter **2103** as described previously.

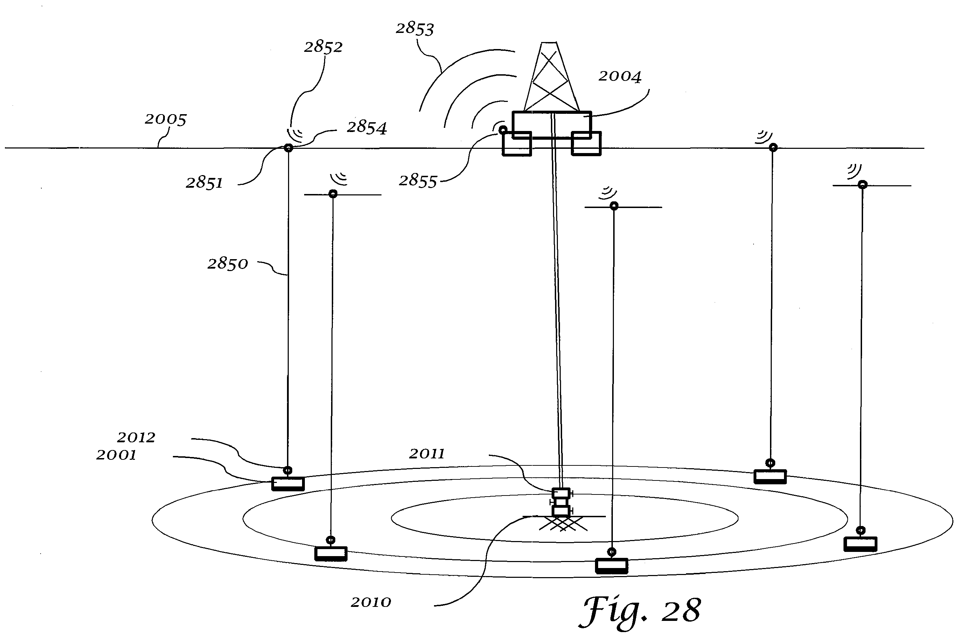

Fig. 28 shows a sampling system that is in standby mode having an additional buoyancy device **2851** which always remains at ocean surface level **2005.** The buoyancy device **2851** includes a surface transmitter/receiver device **2854** that also always remains at ocean surface level **2005,** and is able to transmit and receive a signal **2853**, from a transmitter/receiver **2855** that is located, for example, on the surface facility **2004** and that is in the radio frequency range. This buoyancy device **2851** is connected by a cable **2850** that may or may not be electrically connected to the sampling system **2001** and the sampling deployment buoyancy device **2012.** When a trigger signal **2852** is received, as described in previous embodiments, the device **2851** is able to send a deployment command signal via the cable **2850** to release the sampling deployment buoyancy device **2012**. The buoyancy device **2012** and sampling array **2302** may be physically connected to the cable **2850**, or may deploy independently. When deployed, the buoyancy device **2012** may extend from the ocean floor **2010** to the ocean surface **2005**, or any point in between. In addition, the cable **2850** may also act as a deployment cable for a surface vessel to accurately position the sampling systems on the ocean floor. The cable **2850** may also act as a retrieval cable such that a surface vessel may retrieve sampling systems **2001** that have not yet been deployed, or also to retrieve the sampling array **2302** that has been deployed, or both.

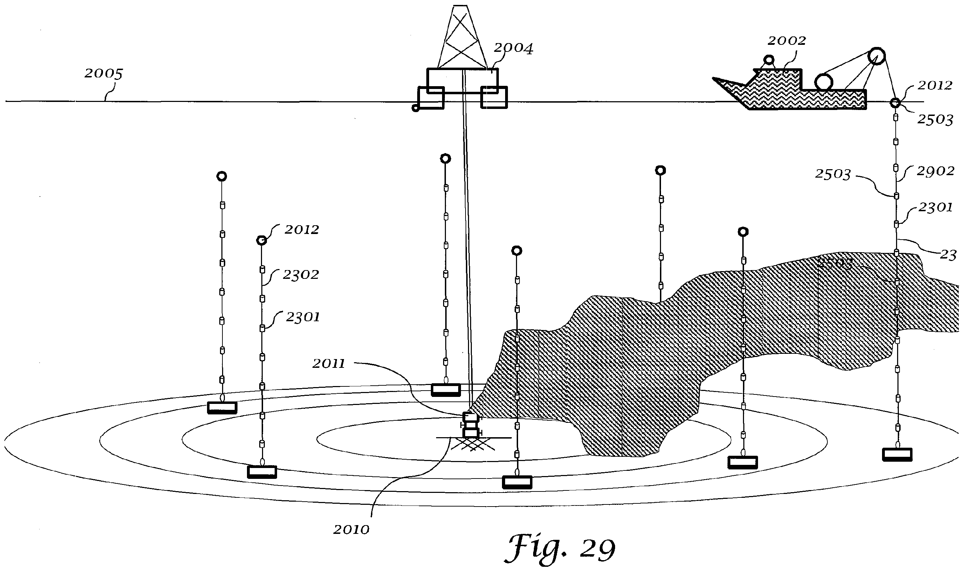

Fig. 29 shows a system that may be deployed for continued monitoring of an offshore leak progression at a perimeter that may be outside of the initial deployment around the site, in accordance with an embodiment of the invention. The system utilizes deployment of "after accident deployment kits" that include one or more sampling systems **2001**. These kits may be ready in standby on-shore for immediate deployment, and may be designed for larger perimeter surveys that are customizable in vertical sampling spacing **2902** for variable resolution. The deployed sampling systems **2001** may extend from ocean floor to surface, or any interval in between. Deployment and retrieval would be done directly from surface using a surface vessel **2002**, with location of the sampling systems **2001** being provided by the surface vessel **2002**, as described in previous embodiments. In addition, the buoyancy device **2012** may be located at the ocean surface **2005** and may include a GPS system for accurate location determination. Additionally at any point or a multitude of points within the sampling array **2302** a positioning device **2503,** as described previously, may be included for accurate prediction of the position of each of the individual sampling arrays or devices over time. The "after accident deployment kits" may be immediately activated by the surface vessel **2002** as soon as they are placed in position for monitoring, or they may be installed on a time delay trigger to start the interval sampling in the future which may be minutes, hours or days.

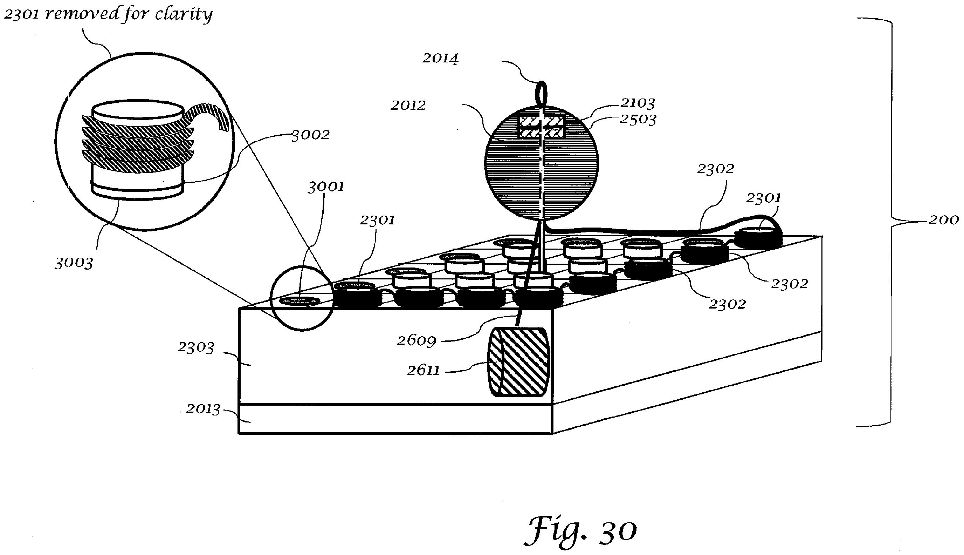

Fig. 30 shows in detail several possible features of the sampling system **2001**, in accordance with an embodiment of the invention. Reference numbers utilized have been described previously. In addition, the sampling system may include any number of sample holder ports **3001** that may be used or left empty as desired, and may vary based on parameters in the area where the system will be deployed. Each sampling device **2301** may be installed into a sample holder port **3001** and may include an O-ring seal **3002**. This seal **3002** may serve to protect the sample inlet ports **3003** which may be, without limitation, located at the bottom of the sampling device **2301**. The sampling device **2301** orientation may be in any direction, but a purpose of the O-ring seal **3002** is to protect the sampling inlet ports from contact with the ocean environment while in standby mode. The purpose of the protection includes avoiding any biofouling or biogrowth occurring at the sample inlet port that may impede sampling or contaminate the acquired sample. The O-ring seal **3002** may be disengaged as each sampling device is pulled out of the containment unit, allowing perfectly clean, unobstructed entry points for the sample fluid to enter the sampling device. The O-ring seal **3002** may provide a full pressure seal to hydrostatic pressure, or may be prefilled with fresh water, or any known fluid that rejects biofouling or biogrowth. This fluid may or may not also be used for hydraulic compensation at the O-ring seal. The system may be configured such that the sample holder ports **3001** automatically fill with seawater at the release of the buoyancy device **2012**, such as by opening a fluid inlet at the release of the buoyancy device.

According to related embodiments of the invention, a sampling system may include location devices such as global positioning systems (GPS units). Said sampling system may be equipped with emitters capable to send the GPS coordinates either via a satellite data link, or via the phone network system or by other means of radio communication. Once it has been released from the ocean floor and has reached the ocean surface, a sampling system may emit radio waves, electromagnetic, acoustic or optical beacon signals, so as to alert ships or other ocean vehicles of its presence and to send current position to a data collection system. The GPS units and communication emitters may be built into, or attached to each sampling unit on the sampling system, and/or to a buoyancy device.

### Monitoring System (Time stamping and sample acquisition monitoring)

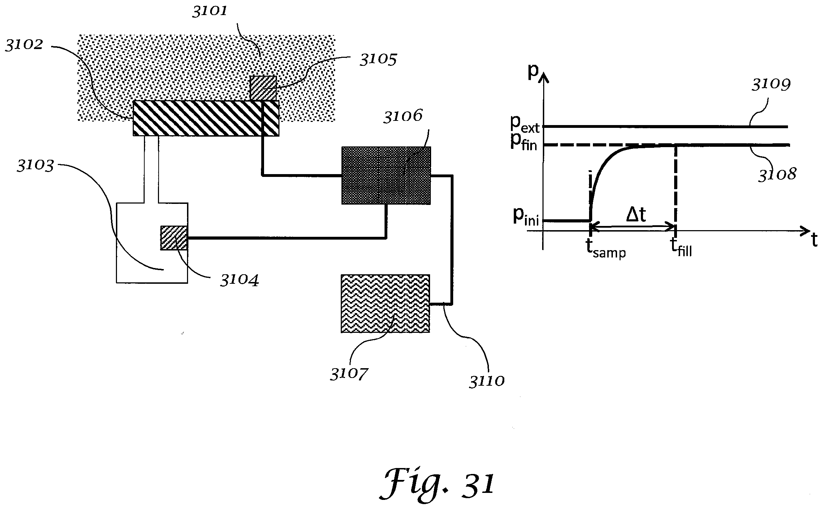

Figure 31 shows a monitoring system for recording sample acquisition time of each sample. More particularly, sample device **3102** includes a pressure active device **3104,** in accordance with an embodiment of the invention. The sample device may be, without limitation, as described in above embodiments, configured to acquire a sample from an external fluid **3101.** The sampling device **3102** includes a sample chamber **3103** for collecting the sample. Said sample chamber **3103** may incorporate in its interior a pressure-active device **3104** that responds to pressure within the sample chamber **3103**. The pressure-active device **3104** may be in communication with a processor **3106** for analyzing and/or recording the signal from said pressure-active device **3104.** The processor **3106** may include an amount of electronic memory in the form of random access memory, flash memory, permanent memory, electrically erasable programmable memory or any other form of electronic memory known in the art. The pressure-active device **3104** may be a pressure sensor, a pressure switch, a pressure gauge, a pressure transducer, or any other device known in the art that can transform a pressure variation into a signal.

The processor **3106** may be in communication with a second pressure-active device 3105 that is placed in and responds to the pressure of the external fluid **3101**. The processor **3106** may further be in communication with a remote system **3107,** to which it may transmit data via a wired or wireless link **3110**.

If the pressure-active device **3104** is a pressure sensor, the processor **3106** may record a pressure curve **3108** of the pressure inside the sampling chamber **3103,** and by analyzing the data from said pressure curve it may calculate the exact moment timestamp (tₛₐₘₚ) of the sample acquisition initiation. The timestamp of the sample acquisition tₛₐₘₚ can be inferred, for example, by monitoring the sample chamber pressure sensor for a significant deviation from the initial pressure in the sample chamber (pᵢₙᵢ).

In various embodiments, the processor **3106** may process the pressure data originating from pressure sensor **3104** so as to determine a sample fill-up duration Δt by recording the time (t_{fill}) when pressure stabilizes within the sample chamber **3103**, and subtracting from this value the time corresponding to beginning of the sample acquisition tsamp: Δt=t_{fill}-tₛₐₘₚ

In various embodiments, the processor **3106** may process the pressure data originating from pressure sensor **3104** to determine the total volume (Vₛₐₘₚ) of the sample acquired. This volume can be inferred by knowing the volume V₀ of the sample chamber **3103** and the value pᵢₙᵢ of the initial pressure in the sample chamber **3103** prior to sample acquisition, the value p_{fin} at which pressure has stabilized in the sample chamber after the sample acquisition, and the pressure pₑₓₜ measured by the pressure sensor **3105** monitoring the pressure of the external medium **3109.**

The processor **3106** may determine Vₛₐₘₚ by using the following formula, which assumes the sample acquisition process as being isothermal and the gas initially contained in the sample chamber to be an ideal gas, in which case Vₛₐₘₚ=V₀(1-pᵢₙᵢ/p_{fin}).

A difference between p_{fin} and pₑₓₜ may be interpreted by processor **3106** as evidence of clogging during the sample acquisition process.

If the pressure-active device **3104** is a pressure switch, the moment of the activation of the pressure switch as recorded by the processor **3106** corresponds to the timestamp of the sample, tₛₐₘₚ.

### Integration of optical elements within or around the sample chamber

According to an embodiment of the present invention, a sample chamber of one of the above-described sampling devices may include optical elements for performing a measurement of, without limitation, turbidity, absorbance, color, transmittance, auto fluorescence, or fluorescence, or any combination thereof.

The sample chamber may incorporate certain optical components, either inside the sample container or in its proximity, in order to assure that the light travels across or around, or otherwise interacts with the sample in an optimal way.

Said sample chamber may be equipped, without limitation, with: one or several optical windows allowing an optical measurement to be performed on the sample contained within the sample chamber, one or multiple light sources, optical detectors, sensors or recording devices (with no limitation: cameras, individual photodiodes or arrays thereof, other types of optical sensors, phototransistors, avalanche photodiodes, photomultipliers), mechanical positioning assemblies, fibers, diaphragms, mirrors, optically absorbing surfaces, optical filters or any other type of optical component or device known to the person skilled in the art, or any combination or configuration thereof.

Data obtained from the optical elements equipping the sample chamber may be used to measure the exact time of the sample acquisition (its timestamp). As an example, a processor could monitor a change in the optical properties of the sample chamber. Said processor may process data from an absorbance measurement performed on the acquired sample at a wavelength where the sample fluid absorbs light (such that the measured absorbance will be higher after sample acquisition than prior to it). Alternatively, said processor may use the presence of an optical signal (or the lack thereof) to infer a deviation of the light path due to a change in optical refraction index that is indicative of a sample being present within the sample chamber.

Any other type of measurement, optical or not, that is known to the person skilled in the art, may be used to determine whether a sample has been acquired within the sample chamber. This may include a conductivity measurement, a temperature measurement, an electrochemical measurement, an optical measurement, a physical measurement, a force measurement, a deflection measurement, a chemical measurement, a biological or biochemical measurement, or any combination thereof.

### Passive timing mechanism of improved precision

As described above in the background section, assume one sample needs to be acquired every hour for a period of twenty four hours. Twenty four sampling devices are deployed at t=0, device numbered n (1<n<24) having a time constant of n hours prior to triggering the acquisition of its corresponding sample. If there is a ten percent error in the fluidic clock of each sampling device, that means that it is likely that the order of the sampling events will be disturbed. For example, the 10th device may acquire its sample at t=11h, and the 11th device at t=10h, thus they will be out of order.

A system is provided that advantageously includes a number n of sampling devices that are being timed by fluidic clocks in such a manner that the timing mechanism of the n+1st device is triggered by the acquisition of the nth sample, in accordance with an embodiment of the invention. Illustratively, in the above-described application, each device could have a time constant of 1hr (or 60 minutes). A ten percent random error in the timing means that the time interval between one sample n and the subsequent one n+1 will carry an absolute error of 6 minutes, but all the samples will be acquired in sequence.

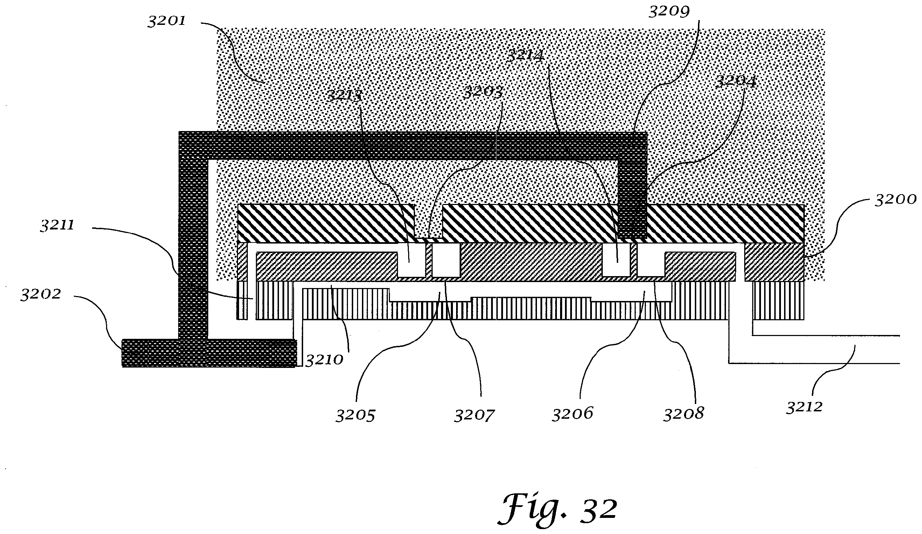

More particularly, Figs. 32-34 show embodiments of the invention that result in passive timing mechanisms of improved precision. A sampling device is provided that includes multiple sampling mechanisms capable of timing and performing the acquisition of multiple (n) samples in an electrically-passive way, where the electrically-passive timing mechanism corresponding to sample i+1 is triggered at a time instant that is related to the time of acquisition of sample i. The sampling device acquires samples from an external fluid.

As shown in Fig. 32, an exemplary sampling device **3200** may include two timing diaphragms **3207** and **3208,** two connected timing cavities **3205** and **3206,** two mechanical structures **3203** and **3204** and two isolated cavities **3213** and **3214.** The first mechanical structure **3203** separates the external fluid **3201** from the first isolated cavity **3213.**

The sampling device **3200** further includes a conduit **3210** that may be a microfluidic channel or a capillary tube, and that may have a predefined geometry. Upon applying pressure to a timing fluid **3202** within the conduit, said timing fluid **3202** being a liquid or a gas, said timing fluid **3202** advances within the conduit **3210** at a speed, for example, that may be dictated by the applied pressure, the predefined channel geometry and known timing fluid properties. The timing fluid conduit **3210** is also connected, by a tube or similar fluidic connection **3209,** to the second mechanical structure **3204.** Upon reaching the connected timing cavities **3205** and **3206** and filling them after a timing interval, the timing fluid **3202** applies pressure to the two timing diaphragms **3207** and **3208** simultaneously, thus destroying their corresponding mechanical structures **3203** and **3204,** for example, by piercing and consequently rupturing and/or collapsing them.

The first mechanical structure **3203,** once destroyed, allows a sample of the external fluid **3201** to be acquired by enabling the external fluid to enter the isolated cavity **3213,** which may then further lead to a sampling chamber via a channel, tube pipe or any other type of fluidic connection **3211.** The second mechanical structure **3204,** once destroyed, opens a passage for the timing fluid **3202** to enter the timing fluid conduit **3212** of a subsequent sampling mechanism, thus acting as an effective trigger for timing the acquisition of the next sample.

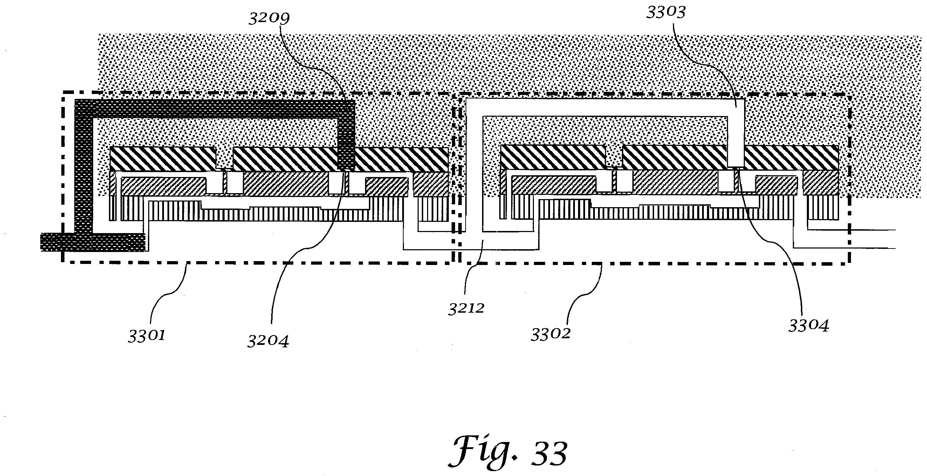

Fig. 33 shows two sampling devices **3301** and **3302,** each as described by Fig. 32, such that the timing fluid conduit **3212** of sampling device **3303** is connected to the second mechanical structure **3204** of sampling device **3301.** The tubes **3303** and **3209** that allow the fluidic communication between the timing fluid and the second mechanical structures **3204** and **3304** of sampling devices **3301** and **3302** may be distinct.

In the configuration shown in Fig. 33, the acquisition of a sample by device **3301** happens simultaneously with the destruction of the mechanical structure **3204,** which allows the timing fluid to enter device **3302** and, following another timing interval, the device **3302** will acquire its own sample. Any number of such sampling devices may be connected in sequence, each sampling device acquiring its sample after being triggered by the previous sampling device.

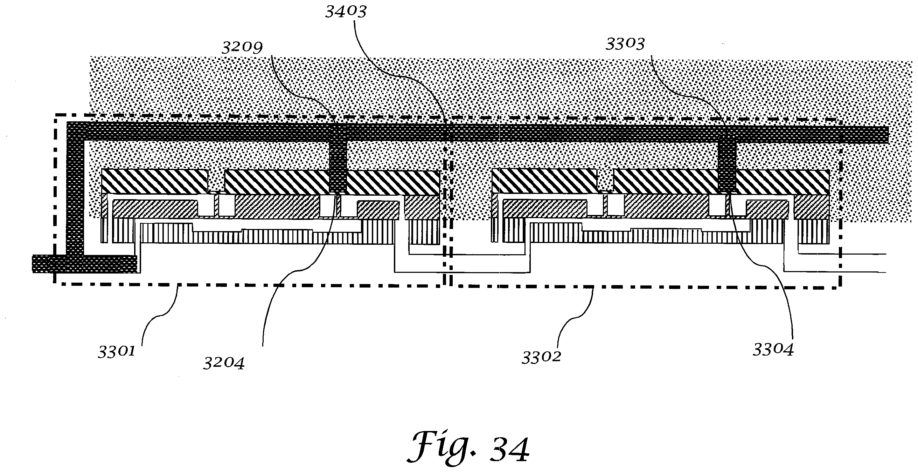

Fig. 34 shows a related embodiment of Fig. 33, showing the same two sampling devices **3301** and **3302,** and further showing the tubes **3303** and **3209** allowing the communication of the timing fluid with the second mechanical structures **3204** and **3304** of the sampling devices **3301** and **3302.** It is further shown that the tubes **3303** and **3209** may be connected using a common manifold **3403** or a similar fluidic connection.

It is understood that such operation can be implemented using other embodiments of the sampling mechanism. For example, using pistons instead of timing diaphragms, as described previously, to pierce the mechanical structures.

### Microbiological measurement

It may be advantageous to allow a sample to incubate for a certain period of time, so as to allow a certain type of microorganism to multiply and grow. According to further embodiments of the present invention, a sampling system may be equipped with a sample chamber that is partially pre-filled with a culture medium. Said culture medium may be selective, allowing only select classes of microorganisms to develop and grow. Said sample chamber may be configured so that, upon sample acquisition, the sample comes in contact with the culture medium. The culture medium is selected such that, if the sample is contaminated with a select class of microorganisms, these will multiply, over a period of time called the incubation period. For example, and without any limitation, certain commercial culture media and bioreagents exist (such as, without limitation, the bio-reagents commercialized under the brand names ReadyCult and Colilert), that allow coliform bacteria to incubate, and over a period of incubation time of several hours the samples change color or fluorescence properties which can lead to the detection and quantification of the said coliform bacteria or of certain classes thereof.

Said sample chamber may include a temperature control mechanism that ensures that the sample temperature is maintained within a range that is optimal for sample incubation.

Said sample chamber may include chemical and/or biological reagents, and/or biocides that react in the presence and/or of the quantity of said microorganisms. Detection of said reaction result may be performed optically, for example (without implying any limitation) by performing an optical absorption measurement, a color measurement, or by monitoring its fluorescence, or its auto-fluorescence.

This embodiment may be combined with other embodiments of the present invention.

### Built-in redundancy

Due to the impracticality of on-line operation monitoring for passive devices such as the above-described devices and systems, it may be advantageous to incorporate various redundancy schemes, to minimize the chance of failure due to unforeseen circumstances. Redundant timing and sensing mechanisms, rendered possible by the extreme miniaturization may be integrated within the device. All critical device components may be built in multiple copies on a single chip, providing parallel fluid and measurement paths in case of failure (e.g., due to channel clogging or sensor malfunction). Single chips may be designed to include multiple sensor chambers for sample analysis, as well as multiple acoustic-emission isolation diaphragms and associated cavities, thus providing multiple assays and hence improved measurement statistics once the devices are recovered at the surface. Multiple timing mechanisms having different time constants may be incorporated onto a single device as well, thus providing a measurement time-series to monitor the evolution of a parameter of interest over a device well injection and retrieval cycle. The resulting device architecture can be extremely robust and should be capable of providing a reliable measurement even in the most adverse environmental conditions.

### Harsh environment compatibility

Completely passive systems represent an advantageous approach to sensing in the very harsh environments specific to the oilfield (e.g., high temperature and pressure (HPHT), corrosive fluids, severely constrained geometry). The above-described embodiments allow the deployment of smart passive devices that are capable of performing a number of specific, well-defined functions in, without limitation, the subterranean environment surrounding an oil well, without requiring power, monitoring, or telemetry. Such smart passive devices can be deployed downhole by pumping along with frac- or other injected fluids, or they can be integrated within existing oilfield measurement tools such as the MDT tool, the FMT tool or the SFT tool. The smart devices may acquire, react with, and isolate a sample of downhole fluid, and, once retrieved from the reservoir, they can be interrogated by optical, electrical or other means to provide information about the environment they have been exposed to (e.g. chemical or physical properties of the fluids encountered) as well as about the times when the measurements were performed. Additionally, as described above, the device can emit bursts of acoustic signals at pre-defined times which can allow device localization by, without limitation, triangulation using multiple microphones.

All the device functionalities recited above may be implemented in multiple applications, and are not limited in any way to oilfield measurements. Examples of different applications include, but are in no way limited to: submarine deployment of such systems as in a body of water, river, lake, sea, ocean; measurements within water wells and aquifers; waste water storage tanks and reservoirs, and the monitoring thereof; and injection wells for carbone dioxide sequestration.

The above-described embodiments are not constrained to a specific sensing technology - several technologies are compatible with and can be integrated within such a smart passive device, such as, without limitation: purely chemical sensors (e.g. titration reactions), corrosion sensors, MEMS sensors, electrochemical sensors, and functionalized nanoparticles. The purely passive devices may be mission-specific so as to integrate only those functions that are absolutely paramount to performing and later interpreting the specific measurement (or chemical reaction) of interest; all additional functionality will be provided externally after recovery. This purely passive approach therefore minimizes the risk of system failure due to environmental issues.

### Ultimate size miniaturization

Besides the capability to survive a harsh environment, a fully passive system provides ultimate miniaturization capabilities. Typically, physical transducers occupy only a very small percentage of the total package size in miniaturized sensors (such as those using MEMS technology), the rest being occupied by electronics and connections. A passive approach eliminates the need to operate electronics down-hole, and thus can lead to impressive size reduction. The use of small, passive devices, that may be fabricated using, without limitation, MEMS technology, permits deployment within pores and/or fractures of the rock. Such deployment may be performed, for example, as part of a proppant formulation during hydraulic fracturing operations.

In summary, the above-described devices enable a variety of functionalities. These functionalities include, without limitation, the following:
1. mechanical protection and hermetic transport of the device within the external environment (by pumping or injection), or deployment within various measurement tools;
2. sample acquisition, material release and/or chemical reaction in-situ at pre-defined times, using passive microfluidic timing mechanisms that may include, without limitation, a diaphragm or a piston;
3. sample isolation from external medium prior to and after acquisition (cross-contamination control);
4. integrated redundancy mechanisms to assure correct device operation even in cases of failure of one of the sample mechanisms;
5. monolythic integration with standard sensor technologies;
6. three-dimensional positioning using coded and/or uncoded acoustic signal emission;
7. external sensor interrogation capability after retrieval at the surface;
8. filtering
9. measuring viscosity of an external fluid;
10. maintaining a sample at high pressure after sample acquisition, to ensure sample integrity and single-phase character;
11. complex sample manipulations, filter backflushing, and transfer between vials;
12. use of a manifold in sampling system
13. chemical /biochemical/microbiological sample measurement(s);
14. integration of optical elements within or around the sample chamber
15. daisy chain configuration of multiple sampling systems;
16. external control of sampling time/ triggered sampling implementation;
17. modifying sampling timing using different passive timing durations (for example, by changing the timing cavity volume);
18. pollution monitoring system/deployment
19. monitoring functionality (for example, time stamping and/or sample acquisition monitoring)

The above-described devices provide robust, highly miniaturized smart passive sample chambers/vessels that can be integrated with several sensor technologies to perform critical in-situ measurements for, without limitation, the oilfield, the ocean, or a living body, or to provide information about the positioning of devices during fluid injection or fracturing operations. One of the main features of the device is its capability to provide a robust timing mechanism to perform, for example, measurements or material release on a pre-defined (or post-inferred) schedule, and/or to emit acoustic signal sequences, which will allow triangulation of the vessel position, thus indicating fluid movement and fracture propagation, within a hydrocarbon reservoir, or other pressurized formation or system. From fracture propagation modeling relative to induced pressures, formation mechanical properties and stress analysis can be performed in-situ. The device may be integrated with standard sensing technologies, allowing a specific measurement or set of measurements to be performed on an isolated fluid sample. The device may also be utilized as part of a proppant formulation during hydraulic fracturing jobs, whereas the passive devices are mixed with slurries and sand grains and are injected alongside into a formation. The device may be used to as a vehicle for time-release of particles, chemical products, or pharmaceutical products. The device may be used in autonomous devices, for example, on robots such as marine remotely-operated underwater vehicles, autonomous underwater vehicles, airborne or ground drones and vehicles, and other types of robotic equipment. The device may be used to monitor flow of external fluids/gases/pollution/contamination in and around, without limitation, cities, chemical plants, nuclear sites, remote regions without power, offshore platforms and other oilfield structures, military missions and battlegrounds.

These combined capabilities result, without limitation, in a very versatile device capable of being implemented within a tool or injected or otherwise deployed in a formation, or living body, or other body of fluid, to provide measurements on samples acquired and/or to release particles, at different locations in, without limitation, an oil reservoir, living body, or other body of fluid, and at multiple times, and to communicate its position via, for example, acoustic emission.

The embodiments of the invention described above are intended to be merely exemplary; numerous variations and modifications will be apparent to those skilled in the art. All such variations and modifications are intended to be within the scope of the present invention. These and other obvious modifications are intended to be covered by the claims that follow.

## Claims

1. A system for acquiring at least one sample from a fluid, the system comprising:
one or more devices (1600); each device (1600) including a sampling mechanism having:
an isolated cavity (1611) that is initially inaccessible to the external fluid (1601);
an electrically passive timing mechanism including a piercing structure (1603), a timing cavity (1616) and a conduit (1615) in fluidic communication with the timing cavity (1616) ; and
a mechanical structure (1602) separating the isolated cavity (1611) from the exterior environment,
the system being **characterized in that**
the electrically passive timing mechanism includes a piston (1604), the piston (1604) configured to move within the isolated cavity (1611), the timing mechanism configured so that upon applying pressure to a timing fluid (1609) within the conduit (1615), said timing fluid (1609) advances within the conduit (1615) and upon reaching the timing cavity (1616) and filling it after a timing interval, the timing fluid (1609) applies pressure to a side of the piston (1604), causing the piston (1604) to advance; and **in that**
the timing mechanism being configured so that at the end of the timing interval the advancement of the piston (1604) causes the piercing structure (1603) to pierce the mechanical structure (1602), bringing the isolated cavity (1611) in contact with the external fluid (1601).

2. The system according to claim 1, wherein the electrically passive timing mechanism further includes a sliding seal (1605), the sliding seal (1605) and the piston (1604) separating the isolated cavity (1611) into two portions that are not in fluid communications.

3. The system according to claim 1 or 2, wherein the piston (1604) includes the piercing structure (1603).

4. The system according to claim 1, wherein the timing interval is predetermined based, at least in part, on geometry of the conduit (1615), volume of the timing cavity (1616), pressure applied to the timing fluid (1609), or timing fluid properties, or any combination thereof.

5. The system according to any one of claims 1 to 4, wherein at least one of the one or more devices (1600) includes a plurality of sampling mechanisms having different timing intervals,
wherein the timing mechanism of each sampling mechanism includes a timing cavity (1616), and a conduit (1615) in fluidic communication with the timing cavity (1616), wherein upon applying pressure to a timing fluid (1609) within the conduit (1615), said timing fluid (1609) advances within the conduit (1615) and fills the timing cavity (1616), the timing interval of each sampling mechanism being determined based, at least in part, on the volume of the timing cavity (1616), and wherein the plurality of sampling mechanisms have different timing cavity volumes.

6. The system according to any one of claims 1 to 5, wherein the sampling mechanism further includes a sampling chamber (1607), for receiving fluid from the isolated cavity.

7. The system according to claim 6, further including a one-way check valve (1606) that allows fluid flow from the isolated cavity (1611) into the sampling chamber (1607).

8. The system according to claim 6, wherein the sampling chamber (1607) is removably coupled to the isolated cavity (1611).

9. The system according to any one of claims 1 to 8, further including a trigger mechanism that activates the passive timing mechanism.

10. The system according to claim 9, wherein at least one of the one or more devices (1600) includes a first sampling mechanism and a second sampling mechanism, and wherein the piercing of the mechanical structure (1602) and acquisition of a sample by the first sampling mechanism acts as a trigger for activating the timing mechanism of the second sampling mechanism.

11. The system according to any one of claims 1 to 10, further including a monitoring system for recording sample acquisition time of each sample.

12. The system according to any one of claims 1 to 11, wherein each device (1600) includes a sampling chamber (1607) for storing an acquired sample, the sampling chamber (1607) at least partially filled with a culture medium, a chemical reagent, a biological reagent, or a biocide, or a combination thereof.

13. A method for acquiring at least one sample from a fluid, the method comprising:
deploying at least one device (1600) in the fluid; each device (1600) including a sampling mechanism having:
an isolated cavity (1611) that is initially inaccessible to the external fluid (1601);
an electrically passive timing mechanism including a piercing structure (1603), a timing cavity (1616), a conduit (1615) in fluidic communication with the timing cavity (1616) ; and
a mechanical structure (1602) separating the isolated cavity (1611) from the exterior environment,
the method being **characterized in that**
the electrically passive timing mechanism includes a piston (1604), the piston (1604) configured to move within the isolated cavity, the timing mechanism configured so that upon applying pressure to the timing fluid (1609) within the conduit (1615), said timing fluid (1609) advances within the conduit (1615) and upon reaching the timing cavity (1616) and filling it after a timing interval, the timing fluid (1609) applies pressure to a side of the piston (1604), causing the piston (1604) to advance, and **in that**
the timing mechanism being configured so that at the end of the timing interval the advancement of the piston (1604) causes the piercing structure (1603) to pierce the mechanical structure, bringing the isolated cavity (1611) in contact with the external fluid (1601).

## Patentansprüche

1. System zum Gewinnen von mindestens einer Probe aus einem Fluid, wobei das System aufweist:
eine oder mehrere Einrichtungen (1600); wobei jede Einrichtung einen Probenentnahmemechanismus aufweist:
einen isolierten Hohlraum (1611), der anfänglich für das externe Fluid (1601) nicht zugänglich ist;
einen elektrisch passiven Zeitsteuermechanismus mit einer Durchstechstruktur (1603), einem Zeitsteuerhohlraum (1616) und einer Leitung (1615), welche in Fluidverbindung mit dem Zeitsteuerhohlraum (1616) ist; und
eine mechanische Struktur (1602), welche den isolierten Hohlraum (1611) von der externen Umgebung separiert,
wobei das System **dadurch gekennzeichnet ist,**
**dass** der elektrisch passive Zeitsteuermechanismus einen Kolben (1604) aufweist, wobei der Kolben (1604) konfiguriert ist, sich innerhalb des isolierten Hohlraums (1611) zu bewegen, wobei der Zeitsteuermechanismus so konfiguriert ist, dass beim Aufbringen von Druck auf ein Zeitsteuerfluid (1609) innerhalb der Leitung (1615) das Zeitsteuerfluid (1609) sich innerhalb der Leitung (1615) vorbewegt und wobei beim Erreichen des Zeitsteuerhohlraums (1616) und Füllen desselben nach einem Zeitsteuerintervall das Zeitsteuerfluid (1609) einen Druck auf eine Seite des Kolbens (1604) ausübt, welcher den Kolben (1604) veranlasst sich vorwärts zu bewegen; und dass
der Zeitsteuermechanismus so konfiguriert ist, dass am Ende des Zeitsteuerintervalls das Vorbewegen des Kolbens (1604) die Durchstechstruktur (1603) veranlasst, die mechanische Struktur (1602) zu durchstechen, wobei der isolierte Hohlraum (1611) in Kontakt mit dem externen Fluid (1601) gebracht wird.

2. System nach Anspruch 1, wobei der elektrisch passive Zeitsteuermechanismus des Weiteren eine Gleitdichtung (1605) aufweist, wobei die Gleitdichtung (1605) und der Kolben (1604) den isolierten Hohlraum (1611) in zwei Bereiche, welche in keiner Fluidverbindung sind, trennt.

3. System nach Anspruch 1 oder 2, wobei der Kolben (1604) die Durchstechstruktur (1603) aufweist.

4. System nach Anspruch 1, wobei das Zeitsteuerintervall vorbestimmt ist basierend zumindest teilweise auf der Geometrie der Leitung (1615), des Volumens des Zeitsteuerhohlraums (1616), des Druckes, welcher auf das Zeitsteuerfluid (1609) wirkt oder Zeitsteuerfluideigenschaften oder jegliche Kombination davon.

5. System nach einem der Ansprüche 1 bis 4, wobei mindestens eine der einen oder mehreren Einrichtungen (1600) eine Vielzahl von Probenentnahmemechanismen mit unterschiedlichen Zeitsteuerintervallen aufweist,
wobei die Zeitsteuermechanismen von jedem Probenentnahmemechanismus einen Zeitsteuerhohlraum (1616) und eine Leitung (1615) in Fluidverbindung mit dem Zeitsteuerhohlraum (1616) aufweisten, wobei beim Aufbringen von Druck auf ein Zeitsteuerfluid (1609) innerhalb der Leitung (1615) das Zeitsteuerfluid (1609) sich innerhalb der Leitung (1615) vorbewegt und den Zeitsteuerhohlraum (1616) füllt, wobei das Zeitsteuerintervall von jedem Probenentnahmemechanismus bestimmt wird basierend auf mindestens teilweise dem Volumens des Zeitsteuerhohlraums (1616) und wobei die Vielzahl von Probenentnahmemechanismen unterschiedliche Zeitsteuerhohlraumvolumen aufweisen.

6. System nach einem der Ansprüche 1 bis 5, wobei der Probenentnahmemechanismus des Weiteren aufweist eine Probenentnahmekammer (1607) zur Aufnahme von Fluid aus dem isolierten Hohlraum.

7. System nach Anspruch 6, des Weiteren aufweisend ein Einweg-Rückschlagventil (1606), welches einen Fluidfluss von dem isolierten Hohlraum (1611) in die Probenentnahmekammer (1607) ermöglicht.

8. System nach Anspruch 6, wobei die Probenentnahmekammer (1607) entfernbar mit dem isolierten Hohlraum (1611) gekoppelt ist.

9. System nach einem der Ansprüche 1 bis 8, des Weiteren aufweisend einen Auslösemechanismus, welcher den passiven Zeitsteuermechanismus aktiviert.

10. System nach Anspruch 9, wobei mindestens eine der ein oder mehreren Einrichtungen (1600) einen ersten Probenentnahmemechanismus und einen zweiten Probeentnahmemechanismus aufweist und wobei das Durchstechen der mechanischen Struktur (1602) und das Gewinnen einer Probe durch den ersten Probenentnahmemechanismus als Auslöser zum Aktivieren des Zeitsteuermechanismus des zweiten Probenentnahmemechanismus agiert.

11. System nach einem der Ansprüche 1 bis 10, des Weiteren aufweisend ein Überwachungssystem zum Aufzeichnen der Probenentnahmezeit für jede Probe.

12. System nach einem der Ansprüche 1 bis 11, wobei jede Einrichtung (1600) eine Probenentnahmekammer (1607) zum Speichern einer gewonnenen Probe aufweist, wobei die Probenentnahmekammer (1607) zumindest teilweise mit einem Kulturmedium, einem chemischen Reagenz, einem biologischen Reagenz oder einem Biozid oder einer Kombination davon gefüllt ist.

13. Verfahren zum Gewinnen von mindestens einer Probe aus einem Fluid, wobei das Verfahren aufweist:
Bereitstellen mindestens einer Einrichtung (1600) in dem Fluid, wobei jede Einrichtung (1600) einen Probenentnahmemechanismus hat, aufweisend:
einen isolierten Hohlraum (1611), welcher anfänglich nicht zugänglich zu dem externen Fluid (1601) ist;
einen elektrisch passiven Zeitsteuermechanismus aufweisend eine Durchstechstruktur (1603), einen Zeitsteuerhohlraum (1616), eine Leitung (1615) in Fluidkommunikation mit dem Zeitsteuerhohlraum (1616) und
eine mechanische Struktur (1602), welche den isolierten Hohlraum (1611) von der externen Umgebung trennt;
wobei das Verfahren ist **dadurch gekennzeichnet, dass**
der elektrisch passive Zeitsteuermechanismus einen Kolben (1604) aufweist, wobei der Kolben (1604) konfiguriert ist um sich innerhalb des isolierten Hohlraums zu bewegen, wobei der Zeitsteuermechanismus konfiguriert ist, dass beim Aufbringen von Druck innerhalb der Leitung (1615) auf das Zeitsteuerfluid (1609), sich das Zeitsteuerfluid (1609) innerhalb der Leitung (1615) nach vorne bewegt und beim Erreichen des Zeitsteuerhohlraums (1616) und Füllen desselben nach einem Zeitsteuerintervall das Zeitsteuerfluid (1609) Druck auf eine Seite des Kolbens (1604) aufbringt, welcher den Kolben (1604) veranlasst sich nach vorne zu bewegen und dass
der Zeitsteuermechanismus so konfiguriert ist, dass zum Ende des Zeitsteuerintervalls das nach-vorne-Bewegen des Kolbens (1604) die Durchstechstruktur (1603) veranlasst, die mechanische Struktur zu durchstechen, wobei der isolierte Hohlraum (1611) in Kontakt mit dem externen Fluid (1601) gebracht wird.

## Revendications

1. Un système d'acquisition d'au moins un échantillon à partir d'un fluide, le système comprenant :
un ou plusieurs dispositifs (1600) ; chaque dispositif (1600) comprend un mécanisme d'échantillonnage ayant :
une cavité isolée (1611) qui est initialement inaccessible au fluide externe (1601) ;
un mécanisme de temporisation électriquement passif comprenant une structure perçante (1603), une cavité de temporisation (1616) et un conduit (1615) en communication fluidique avec la cavité de temporisation (1616) ; et
une structure mécanique (1602) séparant la cavité isolée (1611) de l'environnement extérieur,
le système étant **caractérisé en ce que**
le mécanisme de temporisation électriquement passif comprend un piston (1604), le piston (1604) étant configuré pour se déplacer dans la cavité isolée (1611), le mécanisme de temporisation étant configuré de sorte qu'en appliquant la pression sur un fluide de temporisation (1609) dans le conduit (1615), ledit fluide de temporisation (1609) avance à l'intérieur du conduit (1615) et en atteignant la cavité de temporisation (1616) et en la remplissant après un intervalle de temps, le fluide de temporisation (1609) applique une pression sur un côté du piston (1604), provoquant l'avancée du piston (1604) ; et **en ce que**
le mécanisme de temporisation est configuré de sorte qu'à la fin de l'intervalle de temps de l'avancement du piston (1604) cela entraîne la structure perçante (1603) pour percer la structure mécanique (1602), mettant en relation la cavité isolée (1611) en contact avec le fluide externe (1601).

2. Le système selon la revendication 1, dans lequel le mécanisme de temporisation électriquement passif comprend en outre un joint coulissant (1605), le joint coulissant (1605) et le piston (1604) séparant la cavité isolée (1611) en deux parties qui ne sont pas en communication fluidique.

3. Le système selon la revendication 1 ou 2, dans lequel le piston (1604) comprend la structure perçante (1603).

4. Le système selon la revendication 1, dans lequel l'intervalle de temporisation est prédéterminé en fonction, au moins en partie, de la géométrie du conduit (1615), le volume de la cavité de temporisation (1616), la pression appliquée au fluide de temporisation (1609), ou les propriétés du fluide de temporisation, ou toute combinaison de celui-ci.

5. Le système selon l'une des revendications 1 à 4, dans lequel au moins un ou plusieurs dispositifs (1600) comprend une pluralité de mécanismes d'échantillonnage ayant des intervalles de temps différents,
dans lequel le mécanisme de temporisation de chaque mécanisme d'échantillonnage comprend une cavité de temporisation (1616) et un conduit (1615) en communication fluidique avec la cavité de temporisation (1616), dans laquelle lors de l'application de la pression sur un fluide de temporisation (1609) à l'intérieur le conduit (1615), ledit fluide de temporisation (1609) avance à l'intérieur du conduit (1615) et remplit la cavité de temporisation (1616), l'intervalle de temps de chaque mécanisme d'échantillonnage étant déterminé en fonction, au moins en partie, sur le volume de la cavité de temporisation (1616), et dans lequel la pluralité des mécanismes d'échantillonnage ont différents volumes de cavité de temporisation.

6. Le système selon l'une des revendications 1 à 5, dans lequel le mécanisme d'échantillonnage comprend en outre une chambre d'échantillonnage (1607), pour recevoir le fluide de la cavité isolée.

7. Le système selon la revendication 6, comprenant en outre une vanne de contrôle à sens unique (1606) qui permet le flux de fluide depuis la cavité vers la chambre d'échantillonnage (1607).

8. Le système selon la. revendication 6, dans lequel la chambre d'échantillonnage (1607) est couplée de manière amovible à la cavité isolée (1611).

9. Le système selon l'une des revendications 1 à 8, comprenant en outre un mécanisme de déclenchement qui active le mécanisme de temporisation passif.

10. Le système selon la revendication 9, dans lequel au moins un ou plusieurs dispositifs (1600) comprend un premier mécanisme d'échantillonnage et un deuxième mécanisme d'échantillonnage, et dans lequel le perçage de la structure mécanique (1602) et l'acquisition d'un échantillon par le premier mécanisme d'échantillonnage agit comme un déclencheur pour activer le mécanisme de temporisation du deuxième mécanisme d'échantillonnage.

11. Le système selon l'une des revendications 1 à 10, comprenant en outre un système de surveillance pour l'enregistrement du temps d'acquisition de chaque échantillon.

12. Le système selon l'une des revendications 1 à 11, dans lequel chaque dispositif (1600) comprend une chambre d'échantillonnage (1607) pour stocker un échantillon acquis, la chambre d'échantillonnage (1607) étant au moins partiellement remplie d'un milieu de culture, un réactif chimique, un réactif biologique ou un biocide, ou une combinaison de ceux-ci.

13. Méthode d'acquisition d'au moins un échantillon à partir d'un fluide, la méthode comprenant :
le déploiement d'au moins un dispositif (1600) dans le fluide ; chaque dispositif (1600) comprenant un mécanisme d'échantillonnage ayant :
une cavité isolée (1611) qui est initialement inaccessible au fluide externe (1601) ;
un mécanisme de temporisation électriquement passif comprenant une structure perçante (1603), une cavité de temporisation (1616), un conduit (1615) en communication fluidique avec la cavité de temporisation (1616) ; et
une structure mécanique (1602) séparant la cavité isolée (1611) de l'environnement extérieur,
la méthode étant **caractérisée en ce que**
le mécanisme de temporisation électriquement passif comprend un piston (1604), le piston (1604) étant configuré pour se déplacer dans la cavité isolée, le mécanisme de temporisation étant configuré de sorte qu'en appliquant la pression sur le fluide de temporisation (1609) à l'intérieur du conduit (1615), ledit fluide de temporisation (1609) avance à l'intérieur du conduit (1615) et en atteignant la cavité de temporisation (1616) et en la remplissant après un intervalle de temps, le fluide de temporisation (1609) applique une pression sur un côté du piston (1604), provoquant l'avancée du piston (1604), et **en ce que**
le mécanisme de temporisation est configuré de sorte qu'à la fin de l'intervalle de temporisation, l'avancement du piston (1604) entraîne la structure perçante (1603) pour percer la structure mécanique, mettant en relation la cavité isolée (1611) en contact avec le fluide externe (1601).
